# EUROPEAN PATENT APPLICATION

(11) **EP 4 765 149 A1**
(43) Date of publication of application: **24.06.2026**
(21) Application number: 24222071.3
(22) Date of filing: 20.12.2024
(51) Int. Cl.: G16H 30/40, A61C 13/00

(54) **GENERATING A VISUALIZATION MODEL FOR AN ORAL CAVITY**

(71) Applicant: 3Shape A/S, 1060 Copenhagen K (DK)
(72) Inventor: NIELSEN, Malene Leonora, 1060 Copenhagen K (DK); SARI, Arif Yetkin, 1060 Copenhagen K (DK); QIRKO, Sara, 1060 Copenhagen K (DK); FENOY, Laura Montesdeoca, 1060 Copenhagen K (DK); KARLSON, Gustav, 1060 Copenhagen K (DK); VETERE, Elmiro, 1060 Copenhagen K (DK); TYKHOMYROV, Dmytro, 1060 Copenhagen K (DK); GLERUP, Nana, 1060 Copenhagen K (DK); BONDING, Morten, 1060 Copenhagen K (DK)
(74) Representative: Zacco Denmark A/S

(57) **Abstract**

The present disclosure relates to computer implemented method (1100) for generating a visualization model (110D) of an oral cavity. The method includes obtaining (1102) image data (202) associated with an image (202A) of a patient, determining (1104) oral cavity data (110A) associated with an oral cavity of the patient based on the image data (202), retrieving (1106) a three-dimensional (3D) dental model (106A) and a 3D template gingiva model (106B), and determining (1108) one or more alignment parameters (520A, 532A) for aligning at least one of a first portion of the 3D dental model, or a second portion of the 3D dental model with the dental structure of the oral cavity based on the oral cavity data. The method further includes determining (1110) one or more intensity parameters (110B) for matching a first set of colors of the 3D template gingiva model with a gingiva structure (704) based on the oral cavity data, generating (1112) a set of pixel values (110C) for a second set of colors of the 3D dental model based on the oral cavity data and one or more visualization attributes, and generating the visualization model based on the 3D dental model, the 3D template gingiva model, the one or more alignment parameters, the one or more intensity parameters and the set of pixel values.

## Description

### TECHNICAL FIELD

An example embodiment of the present disclosure generally relates to the generation of a visualization model and more particularly relates to the generation of a visualization model for an oral cavity of a patient.

### BACKGROUND OF THE INVENTION

In the field of dentistry, the accurate representation of a patient's dental structures is essential for effective diagnosis, treatment planning, and the evaluation of postoperative outcomes. Digital models serve as vital tools in this process, providing two-dimensional or three-dimensional depictions of the patient's dental anatomy. These models reflect the actual condition of the patient's teeth, gums, and surrounding oral structures, allowing dental professionals to gain a comprehensive understanding of the patient's oral health and facilitate informed decision-making regarding treatment options.

Conventional digital models have been utilized to provide a representation of the oral cavity, serving as a foundational tool in dental practice. However, these traditional models often exhibit significant limitations in their capabilities. Typically, they are designed to offer only a basic depiction of the oral structures, focusing primarily on the dental anatomy and gingival contours. This restricted scope can lead to an incomplete understanding of the patient's unique oral condition, which is critical for effective treatment planning.

Moreover, conventional digital models frequently fail to capture the details necessary for accurately representing a wide range of dental procedures. For instance, when it comes to cosmetic enhancements such as teeth whitening and veneers, or functional treatments like orthodontic applications and retainers, these models do not provide a realistic visualization of the anticipated outcomes. Similarly, they lack the ability to depict procedural changes related to gum contouring, prophylaxis, and the application of dental sealants. This limitation can create challenges in patient communication, as it may prevent dental professionals from effectively conveying the potential results of various treatments to their patients.

Therefore, there is a need for improved digital models that can accurately represent the true anatomical structures of the oral cavity, enhancing diagnostic precision, treatment planning, and patient-provider communication.

### SUMMARY

A computer system, a method and a computer programmable product are provided. The computer system may allow for generation of a visualization model of an oral cavity.

It is an objective of the present disclosure to provide techniques for computer system for generating a visualization model of an oral cavity of a patient, thereby overcoming the limitations associated with conventional methods for dental restoration.

In one aspect, a computer implemented method for generating a visualization model of an oral cavity is provided. The method includes obtaining image data associated with an image of a patient, wherein the image data is indicative of a face of the patient. The method may include determining oral cavity data associated with the oral cavity of the patient based on the image data. The oral cavity data is associated with a dental structure of the oral cavity and a gingiva structure of the oral cavity. In some embodiments, the method may include retrieving a three-dimensional (3D) dental model of the dental structure and a 3D template gingiva model. In some embodiments, the original gingiva from the image data of the patient is morphed to the new tooth positions. The method further may include determining one or more alignment parameters for aligning at least one of a first portion of the 3D dental model, or a second portion of the 3D dental model with the dental structure of the oral cavity based on the oral cavity data. The method further may include determining one or more intensity parameters for matching a first set of colors of the 3D template gingiva model with the gingiva structure, based on the oral cavity data. In some embodiments, the intensity parameters may be color matching parameters or visual fidelity parameters, i.e. other parameters aiding in showing visual realism. The method further may include generating a set of pixel values for a second set of colors of the 3D dental model based on the oral cavity data and one or more visualization attributes. The one or more visualization attributes are associated with a manipulation of the oral cavity in the image of the patient. The method further may include generating the visualization model of the oral cavity of the patient based on the 3D dental model, the 3D template gingiva model, the one or more alignment parameters, the one or more intensity parameters, and the set of pixel values. The method further may include rendering, on a display, the visualization model of the oral cavity.

In accordance with some example embodiments, the method further may include identifying one or more facial landmark features associated with the face of the patient based on the image data. The method further may include determining inclination data associated with the face based on the image data and the one or more facial landmark features. The method further may include identifying an oral cavity region associated with the oral cavity of the patient based on the image data, the one or more facial landmark features and the inclination data. The method further may include segmenting, using a first machine learning (ML) model, the dental structure of the oral cavity and the gingiva structure of the oral cavity based on the identified oral cavity region. The method further may include generating the oral cavity data based on the segmentation.

In accordance with some example embodiments, the method further may include obtaining scan data associated with an intraoral cavity of the patient. The method further may include generating, using a second ML model, the 3D dental model of the dental structure of the patient. The method further may include storing the 3D dental model of the dental structure. Althernatively, the method may display the 3D dental model on a display without storing the 3D dental model.

In accordance with some example embodiments, the method further may include generating a background image associated with the face of the patient based on the image data. In some embodiments, the background image may be generated based on a facial photograph of the patient. The background image comprises one or more facial characteristics of the face. The one or more facial characteristics are exclusive of the oral cavity, i.e the background image may comprise a facial photograph of the patient with any teeth removed. The method further may include generating a current visualization model of the oral cavity based on the 3D dental model, the 3D template gingiva model, the one or more alignment parameters, the one or more intensity parameters. The method further may include overlaying the current visualization model on the background image. The method further may include segmenting each of the dental structure of the oral cavity and the gingiva structure of the oral cavity based on the overlay. The method further may include generating the visualization model of the oral cavity based on the segmentation and the set of corrected pixel values associated with the 3D dental model. The method further may include overlaying the visualization model on the background image. The method further may include generating an updated image of the face of the patient based on the overlaying and the image data.

In accordance with some example embodiments, the method further may include generating a first segmented image associated with the oral cavity, the first segmented image comprising a first set of pixels associated with the dental structure and a second set of pixels associated with the gingiva structure. A value of each pixel of the first set of pixels is set to a predefined value in the first segmented image. The method further may include identifying a first pixel from the first set of pixels, the first pixel having at least one first adjacent pixel. The at least one first adjacent pixel is associated with the second set of pixels. The method further may include determining a first color average associated with the at least one first adjacent pixel based on a first pixel value associated with each of the at least one first adjacent pixel. In some embodiments, the method may include color correction and pixel sampling based on the original 2D background image. This step aids in matching shadows and colors on the 3D dental model and gingiva model to the background image. The method further may include adding a first normally distributed noise to a first distribution of the first color average. The method further may include generating a first corrected pixel value of the set of corrected pixel values based on the addition. The method further may include adjusting the first pixel based on the first corrected pixel value. The method further may include generating at least a part of the visualization model based on the adjusted first pixel.

In accordance with some example embodiments, the method further may include generating a second segmented image associated with the oral cavity, the second segmented image comprising the first set of pixels associated with the dental structure and the second set of pixels associated with the gingiva structure. A value of each pixel of the second set of pixels is set to the predefined value in the second segmented image. The method further may include identifying a second pixel from the second set of pixels, the second pixel having at least one second adjacent pixel. The at least one second adjacent pixel is associated with the first set of pixels. The method further may include determining a second color average associated with the at least one second adjacent pixel based on a second pixel value associated with each of the at least one second adjacent pixel. The method further may include adding a second normally distributed noise to a second distribution of the second color average. The method further may include generating a second corrected pixel value of the set of corrected pixel values based on the addition. The method further may include adjusting the second pixel based on the second corrected pixel value. The method further may include generating at least a part of the visualization model based on the adjusted second pixel.

In accordance with some example embodiments, for determining the one or more intensity parameters, the method further may include identifying a set of gingiva pixels associated with the gingiva structure based on the oral cavity data. The method further may include identifying a second pixel from the second set of pixels, the second pixel having at least one second adjacent pixel. The at least one second adjacent pixel is associated with the first set of pixels. The method further may include retrieving the 3D template gingiva model. A set of template gingival pixels of the 3D template gingiva model is distributed across a coloring coordinate. The method further may include generating a patient image based on masking the set of gingiva pixels with the set of template gingival pixels. The method further may include generating color map data for each pixel of the set of template gingival pixels based on a value of a corresponding pixel of the set of gingival pixels.

In accordance with some example embodiments, for generating the set of pixel values, the method further may include identifying a set of dental pixels associated with the dental structure based on the oral cavity data. The method further may include determining intensity data associated with the set of dental pixels.In some embodiments, the intensity data is associated with a brightness level of each column of pixels of the set of dental pixels. In other embodiments, the intensity data is associated with an average brightness level of a set of pixels representing at least a part of a tooth. The method further may include normalizing the brightness level of each column of pixels based on a predefined range. The method further may include generating an intensity curve based on the normalization. The method further may include identifying a set of points extending along a predefined direction across the dental structure. The method further may include applying the one or more visualization attributes to one or more pixels of the set of dental pixels lying between each consecutive point of the set of points. The one or more visualization attributes are applied based on brightness value associated with the one or more pixels indicated by the intensity curve.

In accordance with some example embodiments, the first portion of the 3D dental model is associated with an upper jaw and the second portion of the 3D dental model is associated with a lower jaw.

In accordance with some example embodiments, for determining the one or more alignment parameters for the 3D dental model, the method further may include identifying a pair of consecutive teeth in the each of the image and the 3D dental model, wherein the pair of consecutive teeth is associated with a predefined type. The method further may include determining edge data associated with the pair of consecutive teeth in each of the image and the 3D dental model. The method further may include determining centre data associated with each tooth in each of the image and the 3D dental model. The method further may include generating a first overlay of the 3D dental model on the dental structure of the image based on the edge data. The method further may include generating a second overlay of the 3D dental model on the dental structure of the image based on the centre data. The method further may include applying a first transformation function to the first overlay, wherein the first transformation function is applied for maximizing an overlapping surface between the dental structure and the 3D dental model. The method further may include applying at least one of: the first transformation function, or a second transformation function to the second overlay, wherein the second transformation function is applied for minimizing a distance between corresponding centres for each tooth in the dental structure and the 3D dental model. The method further may include determining the one or more alignment parameters for the 3D dental model based on the application of first transformation function to the first overlay, or the application of at least one of the first transformation function, or a second transformation function to the second overlay.

In accordance with some example embodiments, the method further may include identifying a bite type associated with the oral cavity based on the image data and a set of predefined bite types. The method further may include determining the one or more alignment parameters based on the identified bite type.

In accordance with some example embodiments, the method further may include the one or more alignment parameters are associated with at least one of: a rotation of a portion of the 3D dental model in at least one of: X axis, Y axis, or Z axis, or a translation of the portion of the 3D dental model in at least one of: X axis, Y axis, or Z axis, and wherein the portion of the 3D dental model corresponds to one of the first portion or the second portion.

In another aspect, a computer system for generating a visualization model of an oral cavity is provided. The system may include a display, a memory configured to store computer executable instructions, and one or more processors execute the computer executable instructions to generate a 3D model of the intraoral site of the patient. The one or more processors are configured to obtain image data associated with an image of a patient. The image data is indicative of a face of the patient. The one or more processors are further configured to determine oral cavity data associated with the oral cavity of the patient based on the image data, wherein the oral cavity data is associated with a dental structure of the oral cavity and a gingiva structure of the oral cavity. The one or more processors are further configured to retrieve a three-dimensional (3D) dental model of the dental structure and a 3D template gingiva model. The one or more processors are further configured to determine one or more alignment parameters for aligning at least one of a first portion of the 3D dental model, or a second portion of the 3D dental model with the dental structure of the oral cavity based on the oral cavity data. The one or more processors are further configured to determine one or more intensity parameters for matching a first set of colors of the 3D template gingiva model with the gingiva structure, based on the oral cavity data. The one or more processors are further configured to generate a set of pixel values for a second set of colors of the 3D dental model based on the oral cavity data and one or more visualization attributes. The one or more visualization attributes are associated with a manipulation of the oral cavity in the image of the patient. The one or more processors are further configured to generate the visualization model of the oral cavity of the patient based on the 3D dental model, the 3D template gingiva model, the one or more alignment parameters, the one or more intensity parameters, and the set of pixel values. The one or more processors are further configured to render, on the display, the visualization model for the oral cavity.

In accordance with some example embodiments, the one or more processors are further configured to identify a set of dental pixels associated with the dental structure based on the oral cavity data. The one or more processors are further configured to determine intensity data associated with the set of dental pixels. In some embodiments, the intensity data is associated with a brightness level of each column of pixels of the set of dental pixels. The one or more processors are further configured to normalize the brightness level of each column of pixels based on a predefined range. In some embodiments, the intensity data is associated with an brightness level of some or all pixels belonging to each tooth. The one or more processors are further configured to identify a set of points extending along a predefined direction across the dental structure. The one or more processors are further configured to apply the one or more visualization attributes to one or more pixels of the set of dental pixels lying between each consecutive point of the set of points, wherein the one or more visualization attributes are applied based on brightness value associated with the one or more pixels indicated by the intensity curve.

In yet another aspect, a computer programmable product is provided. The computer programmable product comprises instructions, which when executed by a computer, cause the computer to carry out steps of, obtaining image data associated with an image of a patient, wherein the image data is indicative of a face of the patient. The steps include determining oral cavity data associated with an oral cavity of the patient based on the image data. The oral cavity data is associated with a dental structure of the oral cavity and a gingiva structure of the oral cavity. The steps further may include retrieving a three-dimensional (3D) dental model of the dental structure and a 3D template gingiva model. The steps further may include determining one or more alignment parameters for aligning at least one of a first portion of the 3D dental model, or a second portion of the 3D dental model with the dental structure of the oral cavity based on the oral cavity data. The steps further may include determining one or more intensity parameters for matching a first set of colors of the 3D template gingiva model with the gingiva structure, based on the oral cavity data. The steps further may include generating a set of pixel values for a second set of colors of the 3D dental model based on the oral cavity data and one or more visualization attributes. The one or more visualization attributes are associated with a manipulation of the oral cavity in the image of the patient. The steps further may include generating a visualization model for the oral cavity of the patient based on the 3D dental model, the 3D template gingiva model, the one or more alignment parameters, the one or more intensity parameters, and the set of pixel values. The steps further may include rendering, on a display, the visualization model of the oral cavity.

The foregoing summary is illustrative only and is not intended to be in any way limiting. In addition to the illustrative aspects, embodiments, and features described above, further aspects, embodiments, and features will become apparent by reference to the drawings and the following detailed description.

According to the present disclosure, a computer implemented method, a computer system, and a computer programable product for generating a visualization model of an oral cavity are provided. One of the purposes of the present disclosure is to provide an accurate depiction of a modification in the oral cavity.

Conventional techniques for generating a modified image associated with a subject may possess limited capability, that may only provide a limited depiction of a part, such as an oral cavity of the subject to be modified. The oral cavity may include dental structure and gingiva structure. The dental structure refers to the anatomical and functional components of the teeth and surrounding tissues in the oral cavity. To this end, the conventional techniques fail to provide an accurate depiction of various dental processes, such as teeth whitening, veneers, gum contouring, orthodontic applications, retainers, prophylaxis, dental sealants, and so forth, which may be done on the subject. In an example, the conventional systems fail to predict and depict accurate representation of brightness of the teeth after teeth whitening process for the subject in a realistic manner. To this end, the present disclosure provides the computer implemented method for generating a visualization model that accurately predicts and depicts or visualizes the oral cavity of the subject in a realistic manner.

The embodiments of the present disclosure allow for accurate prediction and visualization of dental structures and gingiva structure of an oral cavity for various processes, such as post-operative or corrective processes. The visualization of the dental structures and the gingiva structure is performed using a 3D model that provides information on a sub-surface structure of a dental object, such as a tooth of the patient.

### BRIEF DESCRIPTION OF THE DRAWINGS

The present disclosure is illustrated by way of example and not by way of limitation in the figures of the accompanying drawings, in which the like reference numerals indicate like elements and in which:
FIG. 1 illustrates an exemplary network environment in which a computer system for generating a visualization model of an oral cavity is implemented, in accordance with an example embodiment;
FIG. 2A illustrates a block diagram of exemplary operations for generating the visualization model of the oral cavity, in accordance with an example embodiment;
FIG. 2B illustrates a block diagram of exemplary operations for generating a three-dimensional (3D) dental model of a dental structure, in accordance with an example embodiment;
FIG. 3 illustrates a sequence diagram of exemplary operations for generating oral cavity data of the dental structure, in accordance with an example embodiment;
FIG. 4A, FIG. 4B and FIG. 4C illustrate a block diagram of exemplary operations for determining one or more alignment parameters for an upper jaw, in accordance with an example embodiment;
FIG. 5A illustrate a block diagram of exemplary operations for determining the one or more alignment parameters based on a bite type, in accordance with an example embodiment;
FIG. 5B illustrates a block diagram of exemplary operations for determining the one or more alignment parameters associated with an open bite type, in accordance with an example embodiment;
FIG. 5C illustrates a block diagram of exemplary operations for determining the one or more alignment parameters associated with a close bite type, in accordance with an example embodiment;
FIG. 6A and FIG. 6B collectively illustrate a block diagram for generating a background image of a patient, in accordance with an example embodiment;
FIG. 7A and FIG. 7B illustrate a block diagram of exemplary operations for updating a 3D template gingiva model, in accordance with an example embodiment;
FIG. 8 illustrates a block diagram of exemplary operations for generating a visualized dental model of the patient, in accordance with an example embodiment;
FIG. 9A illustrates a block diagram of exemplary operations for generating a current image of the patient, in accordance with an example embodiment;
FIG. 9B illustrates a block diagram of exemplary operations for generating an updated image of the patient, in accordance with an example embodiment;
FIG. 10 illustrates a block diagram of a computer system for generating the visualization model of the oral cavity data of the patient, in accordance with an example embodiment; and
FIG. 11 illustrates a flowchart of a method for generating the visualization model of the oral cavity of the patient, in accordance with an example embodiment.

### DETAILED DESCRIPTION

In the following description, for purposes of explanation, numerous specific details are set forth in order to provide a thorough understanding of the present disclosure. It will be apparent, however, to one skilled in the art that the present disclosure may be practiced without these specific details. In other instances, systems and methods are shown in block diagram form only in order to avoid obscuring the present disclosure.

Reference in this specification to "one embodiment" or "an embodiment" means that a particular feature, structure, or characteristic described in connection with the embodiment is included in at least one embodiment of the present disclosure. The appearance of the phrase "in one embodiment" in various places in the specification are not necessarily all referring to the same embodiment, nor are separate or alternative embodiments mutually exclusive of other embodiments. Further, the terms "a" and "an" herein do not denote a limitation of quantity, but rather denote the presence of at least one of the referenced items. Moreover, various features are described which may be exhibited by some embodiments and not by others. Similarly, various requirements are described which may be requirements for some embodiments but not for other embodiments.

Some embodiments of the present disclosure will now be described more fully hereinafter with reference to the accompanying drawings, in which some, but not all, embodiments of the disclosure are shown. Indeed, various embodiments of the disclosure may be embodied in many different forms and should not be construed as limited to the embodiments set forth herein; rather, these embodiments are provided so that this disclosure will satisfy applicable legal requirements. Like reference numerals refer to like elements throughout. As used herein, the terms "data," "content," "information," and similar terms may be used interchangeably to refer to data capable of being transmitted, received and/or stored in accordance with embodiments of the present disclosure. Further, the terms "processor", "controller" and "processing circuitry" and similar terms may be used interchangeably to refer to the processor capable of processing information in accordance with embodiments of the present disclosure. Further, the terms "electronic equipment", "electronic devices" and "devices" are used interchangeably to refer to electronic equipment monitored by the system in accordance with embodiments of the present disclosure. Thus, use of any such terms should not be taken to limit the spirit and scope of embodiments of the present disclosure.

The embodiments are described herein for illustrative purposes and are subject to many variations. It is understood that various omissions and substitutions of equivalents are contemplated as circumstances may suggest or render expedient but are intended to cover the application or implementation without departing from the spirit or the scope of the present disclosure. Further, it is to be understood that the phraseology and terminology employed herein are for the purpose of the description and should not be regarded as limiting. Any heading utilized within this description is for convenience only and has no legal or limiting effect.

As used in this specification and claims, the terms "for example" "for instance" and "such as", and the verbs "comprising," "having," "including" and their other verb forms, when used in conjunction with a listing of one or more components or other items, are each to be construed as open ended, meaning that that the listing is not to be considered as excluding other, additional components or items. Other terms are to be construed using their broadest reasonable meaning unless they are used in a context that requires a different interpretation.

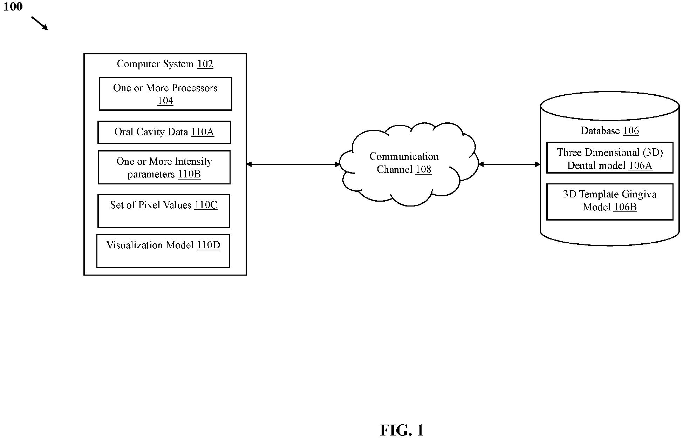

FIG. 1 illustrates an exemplary network environment in which a computer system for generating a visualization model of an oral cavity is implemented, in accordance with an example embodiment. With reference to FIG. 1, an exemplary network environment 100 in which a computer system 102 for generating a visualization model of an oral cavity is implemented is illustrated. For example, the computer system 102 may be used by a user, such as a person having knowledge of dentistry, for example, a user, a dental technician, and so forth. Further, it is possible that one or more components may be rearranged, changed, added, and/or removed without deviating from the scope of the present disclosure.

The computer system 102 may include one or more processors 104 (referred to as the processor 104, hereinafter). The processor 104 is configured to generate the visualization model of the oral cavity of a patient. The network environment 108 may further include a database 106 configured to store data that may be utilized or generated the generation of the visualization model of the oral cavity. The database 106 may be configured to store a three-dimensional (3D) dental model 106A, and a 3D template gingiva model 106B. Further, the network environment 100 may include a communication channel 108 that may be configured to establish communicative coupling between components of the computer system 102, the database 106, and any other external component to which the computer system 102 may connect to.

For example, the processor 104 may be embodied as one or more of various hardware processing means such as a coprocessor, a microprocessor, a controller, a digital signal processor (DSP), a processing element with or without an accompanying DSP, or various other processing circuitry including integrated circuits such as, for example, an ASIC (application specific integrated circuit), an FPGA (field programmable gate array), a microcontroller unit (MCU), a hardware accelerator, a special-purpose computer chip, or the like. As such, in some embodiments, the processor 104 may include one or more processing cores configured to perform independently. A multi-core processor may enable multiprocessing within a single physical package. Additionally, or alternatively, the processor 104 may include one or more processors 104 configured in tandem via the bus to enable independent execution of instructions, pipelining and/or multithreading. Additionally, or alternatively, the processor 104 may include one or more processors capable of processing large volumes of workloads and operations to provide support for big data analysis. In an embodiment, the processor 104 may be in communication with the other components of the computer system 102 via a bus for passing information among components of the computer system 102.

In an example, when the processor 104 are embodied as an executor of software instructions, the instructions may specifically configure the processor 104 to perform the algorithms and/or operations described herein when the instructions are executed. However, in some cases, the processor 104 may be a processor-specific device (for example, a mobile terminal or a fixed computing device) configured to employ an embodiment of the present disclosure by further configuration of the processor 104 by instructions for performing the algorithms and/or operations described herein. The processor 104 may include, among other things, a clock, an arithmetic logic unit (ALU) and logic gates configured to support operation of the processor 104. The network environment, such as network environment 100 may be accessed using the communication channel 108.

Further, the database 106 is an organized collection of structured information, or data, typically stored electronically in a computer system. The database 106 is configured to receive, store, and transmit data. In an embodiment, the database 106 may be configured to transmit the data to the computer system 102. Further, the database 106 may store at least the 3D dental model 106A and the 3D template gingiva model 106B. The 3D dental model 106A may be a 3D model that provides information of a sub-surface structure of a dental object, such as a tooth or a set of teeth of the patient. In an example, the 3D dental model 106A may be generated using an intraoral scanner. Further, the 3D template gingiva model 106B may be a standard template model featuring artificial colors. In an example, the 3D template gingiva model 106B is a standardized anatomical representation of gingival (gum) tissue, designed for use in various applications such as dental education, surgical planning, and prosthetic design.

The communication channel 108 may be wired, wireless, or any combination of wired and wireless communication networks, such as cellular, wireless fidelity (Wi-Fi), internet, local area networks, or the like. In accordance with an embodiment, the communication channel 108 may be one or more wireless full-duplex communication channels. In one embodiment, the communication channel 108 may include one or more networks such as a data network, a wireless network, a telephony network, or any combination thereof. It is contemplated that the data network may be any local area network (LAN), metropolitan area network (MAN), wide area network (WAN), a public data network (e.g., the Internet), short range wireless network, or any other suitable packet-switched network, such as a commercially owned, proprietary packet-switched network, e.g., a proprietary cable or fibre-optic network, and the like, or any combination thereof. In addition, the wireless network may be, for example, a cellular network and may employ various technologies including enhanced data rates for global evolution (EDGE), general packet radio service (GPRS), global system for mobile communications (GSM), Internet protocol multimedia subsystem (IMS), universal mobile telecommunications system (UMTS), etc., as well as any other suitable wireless medium, e.g., worldwide interoperability for microwave access (WiMAX), Long Term Evolution (LTE) networks (for e.g. LTE-Advanced Pro), 5G New Radio networks, ITU-IMT 2020 networks, code division multiple access (CDMA), wideband code division multiple access (WCDMA), wireless fidelity (Wi-Fi), wireless LAN (WLAN), Bluetooth, Internet Protocol (IP) data casting, satellite, mobile ad-hoc network (MANET), and the like, or any combination thereof. The database 108 may be configured to communicate with the processors 106 via the communication channel 108.

The computer system 102 may be configured to store, such as in a memory, data generated by the computer system 102. For example, the computer system 102 may be configured to generate data, such as oral cavity data 110A, one or more intensity parameters 110B, a set of pixel values 110C, and a visualization model 110D.

The oral cavity data 110A may include the information derived from the oral cavity, such as a comprehensive collection of information related to the anatomical, physiological, and pathological aspects of the oral cavity, which may include structures such as the lips, cheeks, gums, teeth, tongue, and palates. Further, the oral cavity data 110A may be associated with a dental structure of the oral cavity and a gingiva structure of the oral cavity of the patient. In an example, the oral cavity data 110A may include information associated with a set of dental pixels associated with the dental structure and a set of gingiva pixels associated with the gingiva structure. The pixels may be smallest discrete unit of a digital image or display, representing a single point in a raster grid and including information for its coordinates within that grid. Each pixel is defined by its color, typically expressed using color models like RGB (red, green, blue), allowing for a wide range of colors based on bit depth. The resolution of an image or screen is determined by the total number of pixels it contains, impacting the clarity and detail of the visual representation.

The one or more intensity parameters 110B may be associated with a set of template gingiva pixels. The set of template gingiva pixels may correspond to pixels of the 3D template gingiva model 106B. Furthermore, the one or more intensity parameters may indicate modified colors linked to one or more pixels of the set of template gingiva pixels of the 3D template gingiva model 106B. The modified colors are based on the gingiva of the patient as identified in the oral cavity. The modified colors are used to update the 3D template gingiva model 106B to align it with the gingiva structure of the patient. The modified colors enable enhanced visualization and analysis of the gingival tissue, facilitating improved diagnostic capabilities and treatment planning in oral health applications.

Further, the set of pixel values 110C may be associated with the 3D dental model 106A. The set of pixel values 110C indicate pixel values, such as colors for the set of dental model pixels of the 3D dental model 106A. In an example, the set of pixel values 110C may correspond to the precise color representation of each pixel of the set of dental model pixels associated with the 3D dental model 106A.

The computer system 102 may be configured to generate the visualization model 110D of the oral cavity. The computer system 102 may utilize the 3D dental model 106A and the 3D template gingiva model 106B to generate the visualization model 110D. The 3D dental model 106A and the 3D template gingiva model 106B may be received from the database 106. In an embodiment, the computer system 102 may utilize the oral cavity data 110A, the one or more intensity parameters 110B, and the set of pixel values 110C, to generate the visualization model 110D.

In operation, the computer system 102 is configured to obtain image data associated with an image of a patient. The image data is indicative of a face of the patient. The computer system 102 may obtain the image data that may be stored in a memory of the computer system 102 or in the database 106. The image may refer to a visual representation of data captured by an image sensor, which converts light signals into digital signals. In an example, the image may be captured by an image sensor of an electronic device mounted on, for example, a vehicle, a satellite, a handheld device, or the like.

In an embodiment, the image data is received from a user device associated with a user of the computer system 102, or a user device of the patient. Examples of the user device may include, but are not limited to, a smartphone, a cellular phone, a mobile phone, a consumer electronic (CE) device, an Internet of Things (IoT) device, a computing device, a mainframe machine, a server, a computer workstation, or the like. The patient may provide their images through a user interface of the user device. The interface may include suitable logic, circuitry, and/or interfaces that are configured to receive the image data.

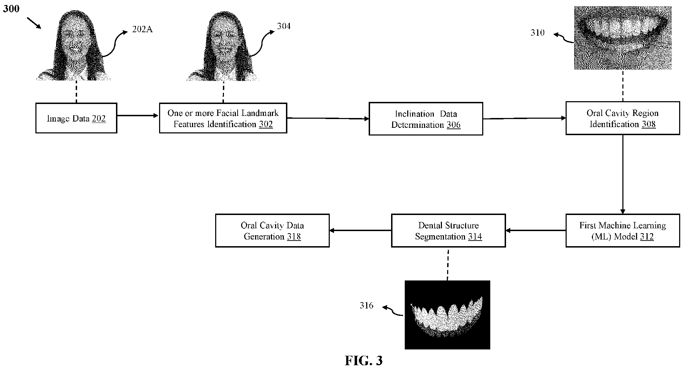

In an embodiment, the computer system 102 is configured to determine oral cavity data 110A associated with the oral cavity of the patient. The oral cavity data 110A is determined based on the image data. The oral cavity data 110A is associated with the dental structure of the oral cavity and the gingiva structure of the oral cavity. In an example, the oral cavity data 110A may include information associated with the set of dental pixels associated with the dental structure and the set of gingiva pixels associated with the gingiva structure. Furthermore, the oral cavity data 110A may be generated using various imaging techniques and computational methods. In an example, the computer system 102 may generate the oral cavity data 110A by applying a segmentation algorithm on a recognized oral cavity of the patient in the image. Details associated with determining the oral cavity data 110A are further described in conjunction with, for example, FIG. 3.

In an embodiment, the computer system 102 is configured to receive the 3D dental model 106A of the dental structure and the 3D template gingiva model 106B. The 3D dental model 106A may be the 3D model that provides information of the sub-surface structure of a dental object, such as a tooth or a set of teeth of the patient. Further, the 3D template gingiva model 106B may be the standard template featuring artificial colors. The computer system 102 may receive the 3D dental model 106A of the dental structure and the 3D template gingiva model 106B from the database 106 via the communication channel 108.

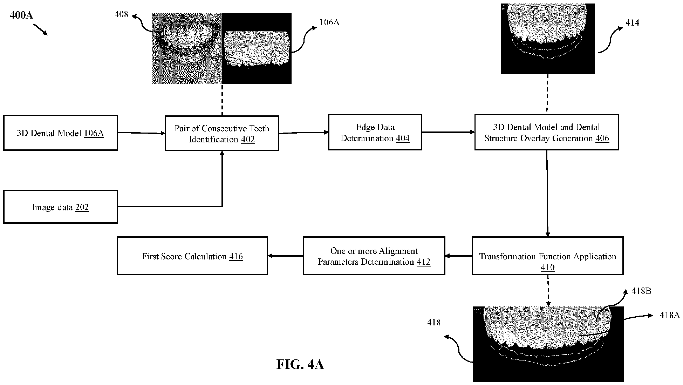

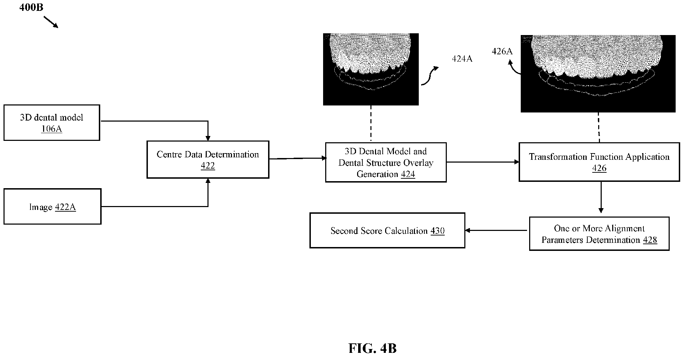

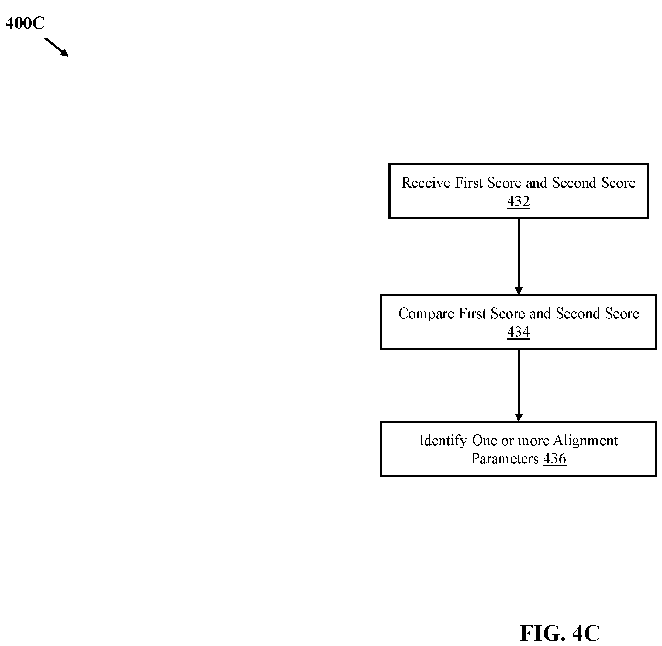

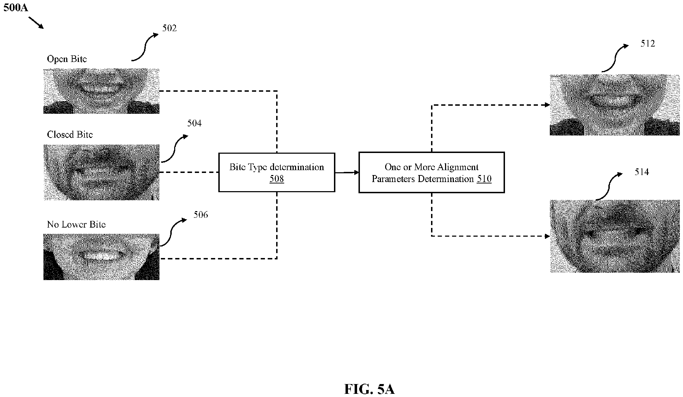

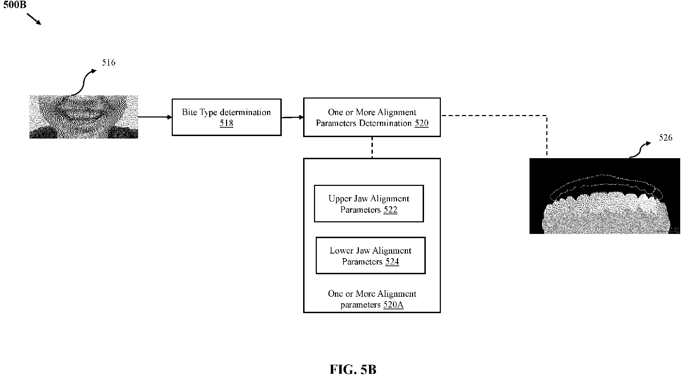

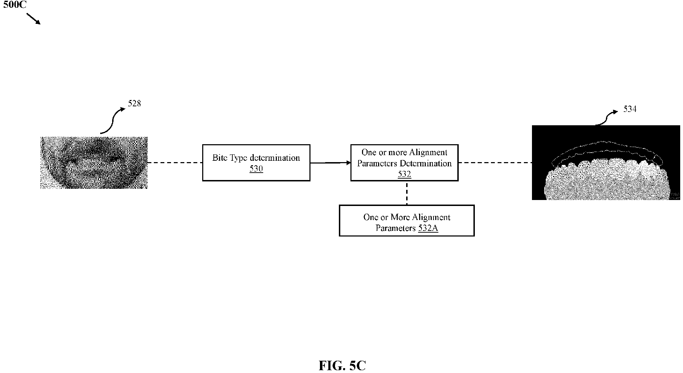

Further, the computer system 102 is configured to determine the one or more alignment parameters for aligning at least one of: a first portion of the 3D dental model, or a second portion of the 3D dental model with the dental structure of the oral cavity. The computer system 102 may determine the alignment parameters based on the oral cavity data 110A. In an example, the first portion of the 3D dental model 106A may correspond to an upper jaw. Similarly, the second portion of the 3D dental model 106A may correspond to a lower jaw. In an embodiment, the computer system 102 may align the upper jaw and the lower jaw of the 3D dental model 106A with the dental structure of the patient based on the one or more alignment parameters. The one or more alignment parameters are associated with at least a rotation of a portion of the 3D dental model in at least X axis, Y axis, or Z axis, or a translation of the portion of the 3D dental model in at least X axis, Y axis, or Z axis. Details associated with determining the one or more alignment parameters are further described in conjunction with, for example, FIG. 4A, FIG. 4B, FIG. 4C, FIG. 5A, FIG. 5B, and FIG. 5C. In some embodiments, the alignment may also comprise determining parameters associated with a virtual camera. For example, when aligning the 3D dental model with the 2D image data, a further alignment parameter may be the field of view of the virtual camera, such that the 3D dental model is correctly overlaid the 2D image data.

In an embodiment, the computer system 102 may be configured to determine the one or more intensity parameters 110B for matching a first set of colors of the 3D template gingiva model 106B with the gingiva structure. The first set of colors are unique artificial colors associated with the set of template gingiva pixels of the 3D template gingiva model 106B. The computer system 102 may be configured to determine the one or more intensity parameters 110B based on the oral cavity data 110A. The one or more intensity parameters 110B may be a set of values indicating an actual color of the gingiva structure.

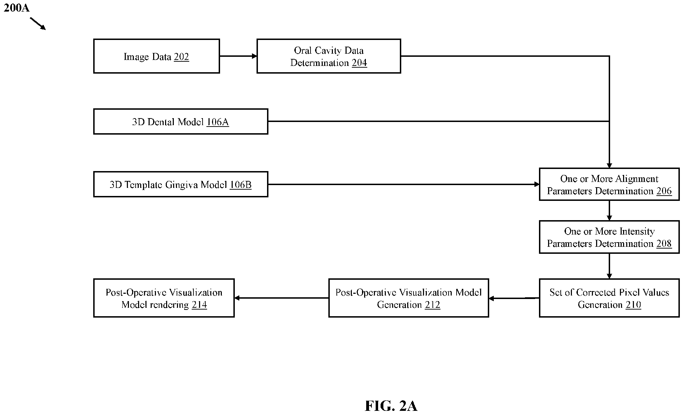

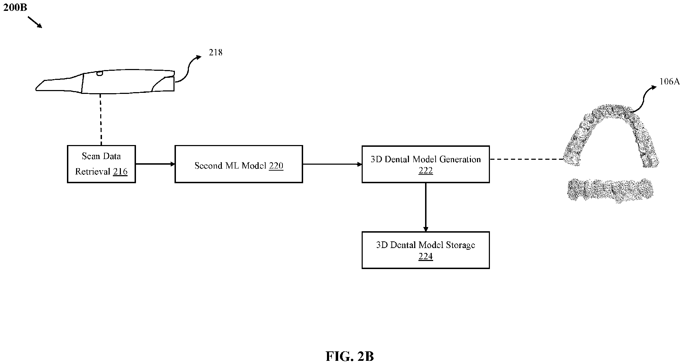

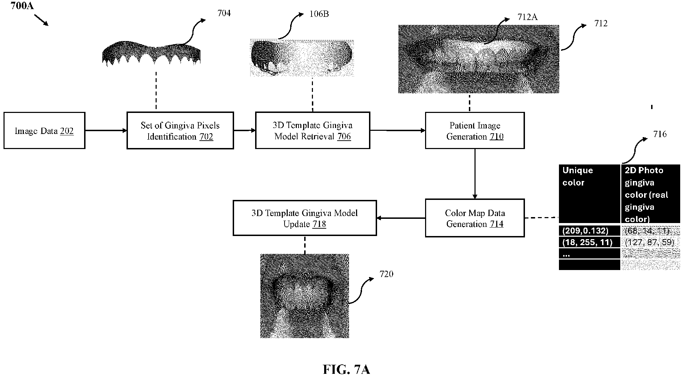

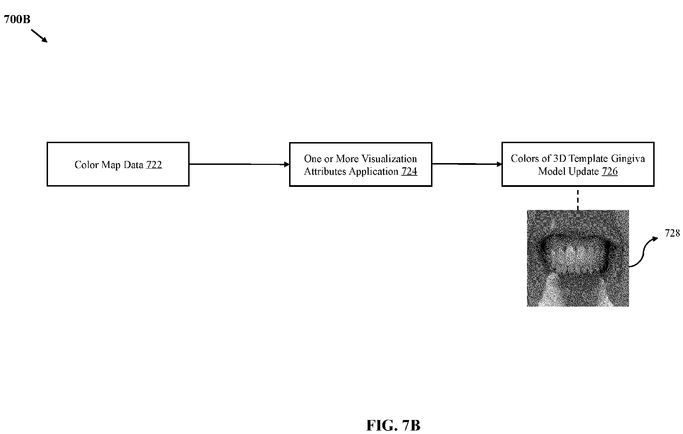

In an embodiment, the computer system 102 is configured to map the first set of colors with the set of gingiva pixels associated with the gingiva structure. Further, the computer system 102 may determine the one or more intensity parameters 110B based on the mapping. Further, the computer system 102 may match each color of the first set of colors of the 3D template gingiva model 106B with the corresponding set of gingiva pixels based on the one or more intensity parameters 110B. Details associated with determining the one or more intensity parameters are further described in conjunction with, for example, FIG. 2, FIG. 7A and FIG. 7B.

In an embodiment, the computer system 102 may be configured to generate a set of pixel values 110C for a second set of colors of the 3D dental model 106A. The second set of colors may be associated with the set of dental model pixels of the 3D dental model 106A. The computer system 102 may generate the set of pixel values 110C based on the oral cavity data 110A and one or more visualization attributes. In an embodiment, the computer system 102 may utilize one or more process to generate the set of pixel values 110C. The set of pixel values 110C indicate values for a visualized dental model, such as a post-operative dental model of the patient. The visualized dental model may be associated with the visualization model 110D. The computer system 102 may correct each color of the second set of colors using the set of pixel values 110C to generate the visualized dental model.

Further, the one or more visualization attributes are associated with a manipulation of the oral cavity in the image of the patient. The one or more visualization attributes are associated with an operative process for the modification of the oral cavity. In an example, the operative process may include teeth whiting, veneers, gum contouring, orthodontic applications, retainers, prophylaxis, dental sealants, and the like. In an example, upon the application of the operative process a modified colors associated with the dental structure may be generated. The one or more visualization attributes may be parameter associated with modified colors of the dental structure generated during the operative process. The one or more visualization attributes may be associated with the process to generate the set of pixel values 110C.

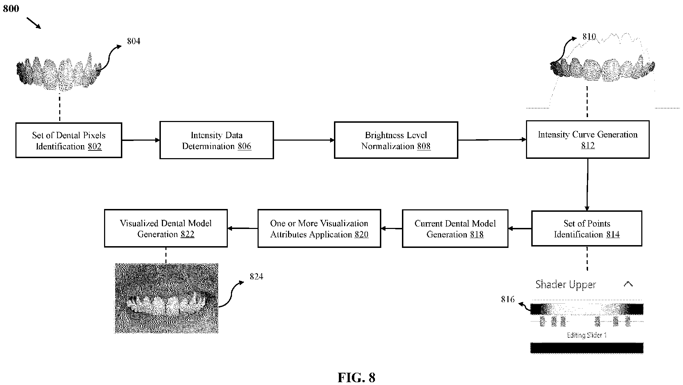

In an embodiment, the computer system 102 may utilize the oral cavity data 110A to generate a current dental model associated with the current visualization model. The current dental model may be a 3D model of the dental structure that may indicate the current condition of the dental structure of the patient before the operative process. Further, the computer system 102 may utilize the one or more visualization attributes and the current dental model to generate the set of pixel values 110C. The set of pixel values 110C may be associated with the visualized dental model of the visualization model 110D. Details associated with generating the set of pixel values 110C are further described in conjunction with, for example, FIG. 8.

Further, the computer system 102 may be configured to generate the visualization model 110D for the oral cavity of the patient based on the 3D dental model, the 3D template gingiva model, the one or more alignment parameters, the one or more intensity parameters 110B, and the set of pixel values 110C. In an embodiment, the computer system 102 may align the 3D dental model 106A with the oral cavity of the patient using the one or more alignment parameters. The computer system 102 may utilize the one or more intensity parameters 110B to generate the visualized gingiva model for the visualization model 110D. Further, the computer system 102 may utilize the set of pixel values 110C to generate the visualized dental model for the visualization model 110D. The operations associated with the generation the visualization model 110D are further explained in conjunction with FIG. 2- FIG. 9.

To this end, the computer system 102 is configured to output the visualization model 110D associated with the oral cavity of the patient. In an example, the computer system 102 may output the visualization model 110D through a user interface of a user device.

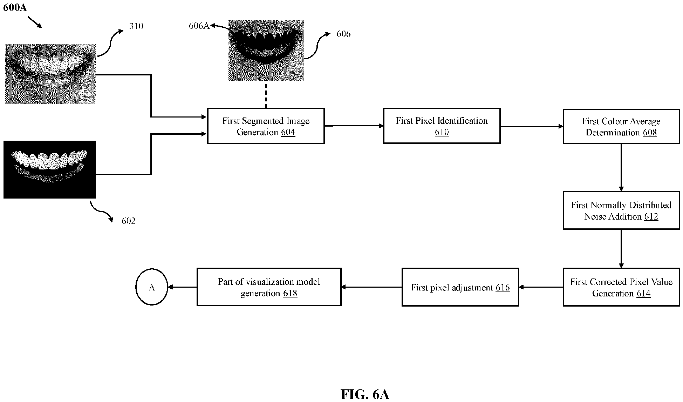

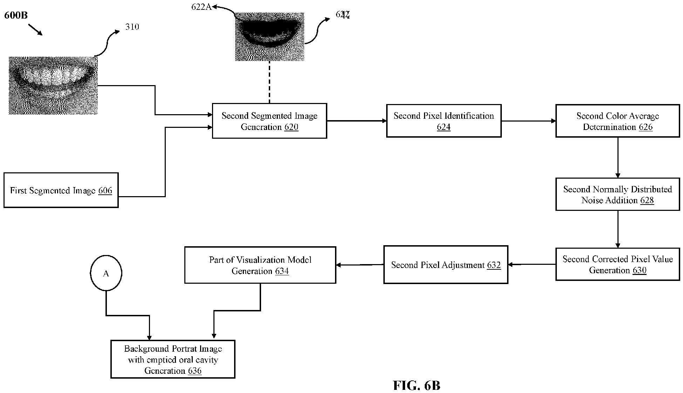

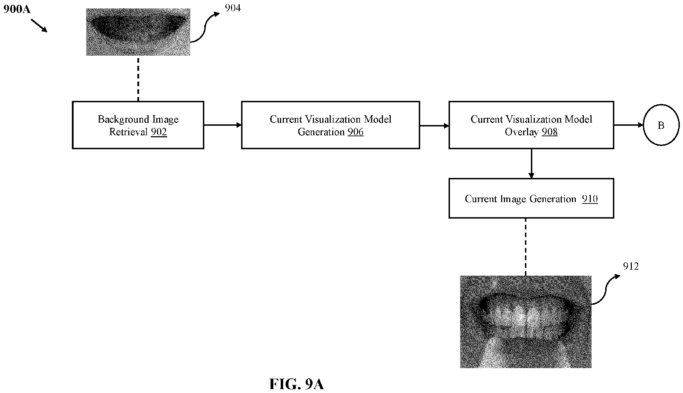

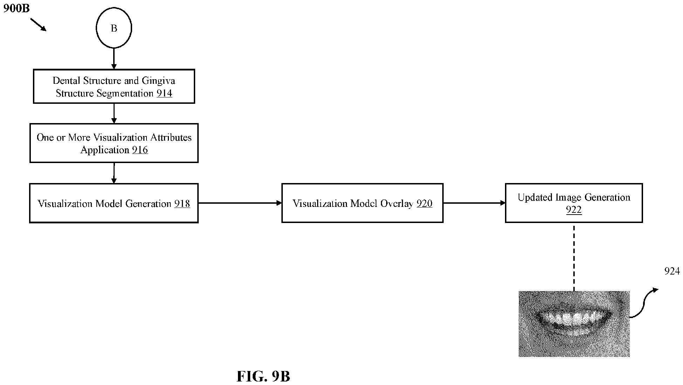

In an example, the computer system 102 may utilize the image of the patient to create an updated image of the patient. The computer system 102 may create a background image of the patient using the image. In an example, the background image may be associated with only the inner lining of the oral cavity of the patient. Further, the computer system 102 may superimpose the visualization model on the background image to generate the updated image of the patient. To this end, the computer system 102 may output the updated image through the user interface. The operations related to the background image generation are explained in conjunction with, for example, FIG.6A and FIG.6B. The operations related to the generation of the updated image are explained in conjunction with, for example, FIG. 9A and FIG. 9B.

FIG. 2 illustrates a block diagram of exemplary operations for generating the visualization model 110D of the oral cavity in accordance with an example embodiment. FIG. 2 is explained in conjunction with elements of FIG. 1.

In an embodiment, the computer system 102 may be configured to receive image data 202. The image data 202 may be associated with the image of the patient. In an example, the image data is indicative of the face of the patient. Further, the image associated with the image data may be a two-dimensional (2D) image. The image may be indicative of the oral cavity associated with the face of the patient. In an embodiment, the computer system 102 may receive the image data from the database 106. In an alternate embodiment, the patient may provide his or her image through a user interface of a user device.

At 204, an oral cavity data determination operation is performed. The computer system 102 is configured to determine the oral cavity data 110A based on the image data 202. In an embodiment, the computer system 102 may apply an image segmentation algorithm to generate the oral cavity data 110A. The image segmentation algorithm may be a computational method used to partition an image or a dataset into distinct regions or segments that share similar characteristics. In an example, the image segmentation algorithm is utilized to identify and isolate specific features or structures within the image, allowing for further analysis. Here, the image segmentation algorithm may be utilized to identify different regions associated with the oral cavity, such as different dental objects associated with the dental structure. In an example, the oral cavity data 110A may include information associated with the set of dental pixels associated with the dental structure and the set of gingiva pixels associated with the gingiva structure.

Furthermore, the computer system 102 may receive the 3D dental model 106A and the 3D template gingiva model 106B from the database 106. In an example, the 3D dental model 106A may be generated using an intraoral scanner. The intraoral scanner may be a dental scanning device that may be used to capture detailed digital impressions of a patient's oral cavity, including teeth, gums, and other structures. Further, the 3D template gingiva model 106B may be a standard template featuring artificial colors. In an example, the 3D dental model 106A, the 3D template gingiva model 106B, and the image data 202 may be associated with the patient.

At 206, a one or more alignment parameters determination operation is performed. In the one or more alignment parameters determination operation, the computer system 102 is configured to determine the one or more alignment parameters based on the oral cavity data 110A. Further, based on the one or more alignment parameters the first portion of the 3D dental model and/or the second portion of the 3D dental model may be aligned with the dental structure of the patient in the image. Further, the first portion of the 3D dental model 106A may correspond to the upper jaw of the dental structure. Similarly, the second portion of the 3D dental model 106A may correspond to the lower jaw of the dental structure.

In an embodiment, the computer system 102 may be configured to identify a bite type associated with the oral cavity. The bite type may be identified based on the image data and a set of predefined bite types. Specifically, the bite type may be generated based on the oral cavity data generated based on the image data. The set of predefined bite types may include, for example, an open bite type, a close bite type, and a no-lower bite type.

In an embodiment, the computer system 102 is configured to determine the one or more alignment parameters based on the identified bite type. In an example, the one or more alignment parameters are associated with the open bite type. In such a case, the one or more alignment parameters are associated with both the upper jaw and the lower jaw. Further the one or more alignment parameters may be associated with at least the rotation of the portion of the 3D dental model in at least X axis, Y axis, or Z axis, or a translation of the portion of the 3D dental model in at least X axis, Y axis, or Z axis. In an embodiment, the one or more alignment parameters may be associated with values that represent the coordinates to align the lower jaw and the upper jaw.

Further, the computer system 102 may align the upper jaw and the lower jaw with the dental structure based on the determined one or more alignment parameters. Further, the operations related to the alignment are explained in conjunction with the FIG. 4A, FIG. 4B, FIG. 4C, FIG. 5A, FIG. 4B, and FIG. 5C.

At 208, a one or more intensity parameters determination operation is performed. In the one or more intensity parameters determination operation, the computer system 102 may be configured to determine the one or more intensity parameters 110B based on the oral cavity data 110A. The one or more intensity parameters 110B are associated with the set of template gingiva pixels of the 3D template gingiva model 106B.

In an embodiment, the computer system 102 may identify the first set of colors associated with the 3D template gingiva model 106B. Further, the computer system 102 may receive information about the set of gingiva pixels of the gingiva structure using the oral cavity data 110A. The computer system 102 may map a color of the first set of colors with each gingiva pixel of the set of gingiva pixels. Further, the computer system 102 may utilize the mapping to identify the one or more intensity parameters 110B. In an example, the computer system 102 may identify the one or more intensity parameters 110B based on the mapping of a unique color of the first set of colors of a pixel of the 3D template gingiva model 106B with a real color of corresponding pixel of the gingiva structure. In an example, the computer system 102 may search the real color in a dictionary. If the real color is present in the dictionary, the real color from the dictionary is used to replace the unique color of the pixel of the 3D template gingiva model. If the real color is not found in the dictionary, a closest color to the real color is found and used for replacing the unique color of the pixel of the 3D template gingiva model. In such a case, if the identified closest color in the dictionary is close to the real color, then the identified closest color is used for replacing the unique color of the pixel of the 3D template gingiva model. However, if a difference between the real color and the closest color in the dictionary is greater than a threshold, an average of one or more real gingiva colors corresponding to one or more pixels near the pixel under consideration is determined and used to replace the unique color of the pixel of the 3D template gingiva model.

Further, based on the one or more intensity parameters 110B, modified colors associated with the 3D template gingiva model 106B may be identified. The computer system 102 may be configured to generate a current gingiva model associated with a current visualization model based on the modified colors. The current visualization model may be associated with a 3D model before the operative process is applied for the modification of the oral cavity. In an alternate embodiment, the computer system 102 may utilize the one or more visualization attributes to generate the visualized gingiva model associated with the visualization model 110D.

At 210, a set of pixel values generation operation is performed. In the set of pixel values generation operation, the computer system 102 may be configured to generate the set of pixel values 110C based on the oral cavity data 110A and the one or more visualization attributes. In an embodiment, the second set of colors may be associated with the set of dental model pixels of the 3D dental model 106A.

Initially, the computer system 102 may utilize the oral cavity data 110A to generate the current dental model associated with the current visualization model. In an example, the computer system 102 may apply one or more operations on the oral cavity data 110A to generate modified pixel values associated with the current dental model. Based on the modified pixel values the current dental model is generated. The operations associated with the current dental model generation operation are further explained in FIG. 8.

Further, the computer system 102 may utilize the one or more visualization attribute to generate the visualized dental model. The one or more visualization attributes may be associated with the operative process for the modification of the oral cavity. Based on the one or more visualization attributes and the modified pixel values, the computer system 102 may generate the visualized dental model.

In an example, the computer system 102 may generate the visualized dental model using the operative process associated with teeth whitening. Further, based on the teeth whitening operative process, a visualization attribute of the one or more visualization attribute may be identified. Further, based on the visualization attributes and the modified pixel values associated with the current dental model, the set of pixel values 110C are determined. At the end, utilizing the set of pixel values 110C, the visualized dental model may be generated.

At 212, a visualization model generation operation is performed. In the visualization model generation operation, the computer system 102 may be configured to generate the visualization model 110D based on the 3D dental model 106A, the 3D template gingiva model 106B, the one or more alignment parameters, the one or more intensity parameters 110B, and the set of pixel values 110C.

At 214, a visualization model render operation is performed. In the visualization model generation operation, the computer system 102 may be configured to render the visualization model 110D of the oral cavity. In an embodiment, the computer system 102 may utilize the image of the patient to create the updated image of the patient based on the visualization model 110D. In the end, the computer system 102 may output the updated image through the user interface.

FIG. 2B illustrates a block diagram 200B of exemplary operations for generating a 3D dental model 106A of a dental structure of the patient, in accordance with an example embodiment. With reference to FIG. 2B there is shown an intraoral scanner 218, a second machine learning (ML) model 220, and the 3D dental model 106A. FIG. 2B is explained in conjunction with elements of FIG. 1 and FIG. 2A.

At 216, a scan data retrieval operation is performed. In an embodiment, the computer system 102 may be configured to receive the scan data. Further, the scan data may be associated with an intraoral cavity of the patient. In an example, the scan data may be obtained using the intraoral scanner 218. The intraoral scanner 218 may be a dental device that may be used to capture detailed digital impressions of a patient's oral cavity, including teeth, gums, and other structures. In an example, the intraoral scanner 218 may include light sources that may project light rays onto an object to be scanned, such as teeth, gums, and other intraoral structures inside the oral cavity of the subject. In an example, the intraoral scanner 218 may be configured to capture a plurality of 2D images during a scanning session of the oral cavity of the patient. The plurality of 2D images may include the plurality of 2D infrared (IR) images indicating images of a dental object of the patient captured using light that is emitted at a wavelength corresponding to a range of while light, red light, green light, IR or near-infrared (NIR) wavelengths. The plurality of 2D IR images may include images of the dental object of the patient from various viewing angles or viewing points.

In an embodiment, the intraoral scanner 218 may be connected with the database 106 via the communication channel 108. Further, the scan data from the intraoral scanner 218 may be stored in the database 106.

The second ML model 220 may receive the scan data. The second ML model 220 may be a computational framework that utilizes algorithms and statistical techniques to analyse and interpret data, enabling it to learn from patterns and make informed predictions or decisions without being explicitly programmed for each specific task. In an embodiment, the computer system 102 may utilize the second ML model 220 to generate the 3D dental model 106A of the intraoral cavity of the patient.

At 222, a 3D dental model generation operation is performed. In the 3D dental model generation operation, the computer system 102 may be configured to generate the 3D dental model 106A using the second ML model 220. Further, the second ML model 220 may generate the 3D dental model 106A using the scan data.

In an embodiment, the second ML model 220 may utilize a 3D segmentation algorithm to generate the 3D dental model 106A. The 3D segmentation algorithm may be designed to accurately segment and reconstruct the 3D dental model 106A of the intraoral cavity from imaging data, such as cone beam computed tomography (CBCT) or intraoral scans. This algorithm utilizes advanced deep-learning techniques to identify and delineate various anatomical structures within the intraoral cavity, providing precise segmentation for dental applications. Further, a person skilled in the art may utilize the operations of the 3D segmentation for constructing the 3D dental model 106A, and details of the same are not described herein for brevity.

At 224, a 3D dental model storage operation is performed. In the 3D dental model storage operation, the computer system 102 may be configured to store the 3D dental model 106A in the database 106 via the communication channel 108. In an alternate embodiment, the computer system 102 may be configured to store the 3D dental model 106A on a server. The server may include suitable logic, circuitry, interfaces, and/or code that stores the first set of rules. The server can be implemented as a cloud server and may execute operations through web applications, cloud applications, HTTP requests, repository operations, file transfer, and the like. Various example implementations of the server include but are not limited to, a database server, a file server, a web server, a media server, an application server, a mainframe server, or a cloud computing server.

In an embodiment of the disclosure, the server is implemented as a plurality of distributed cloud-based resources by use of several technologies that are well known to those ordinarily skilled in the art. A person with ordinary skill in the art will understand that the scope of the disclosure may not be limited to the implementation of the server and the computer system 102 as two separate entities. In certain embodiments, the functionalities of the server can be incorporated in its entirety or at least partially in the computer system 102, without a departure from the scope of the disclosure.

FIG. 3 illustrates a sequence diagram 300 of exemplary operations for generating the oral cavity data of a dental structure, in accordance with an example embodiment of the present disclosure. With reference to FIG. 3 there is shown an image 202A, a facial landmark image, an oral cavity region 310, a 2D segmented teeth 316, and a first ML Model 312. FIG. 3 is explained in conjunction with elements of FIG. 1, FIG. 2A, and FIG. 2B.

In an embodiment, the processor 104 of the computer system 102 may receive the image data 202. In an example, the image data 202 may be received from the memory of the computer system 102. In an example, the computer system 102 may receive the image data 202 associated with an image 202A of the face of the patient. Further, the image 202A associated with the image data may be a two-dimensional (2D) image. The image 202A may be indicative of an oral cavity associated with the face of the patient.

At 302, a facial landmark features identification operation is performed. In the facial landmark features identification operation, the processor 104 is configured to identify one or more facial landmark features associated with the face of the patient. The processor 104 may identify the one or more facial landmarks based on the image data 202. The one or more facial landmarks are specific geometrically significant points on the face of the patient that serve as reference markers for the analysis and interpretation of facial features and expressions of the face. In an example, these landmarks are typically identified based on anatomical structures and may include key points such as the oral cavity, a tip of a nose, inner and outer corners of the eyes, corners of mouth, highest points of eyebrows, chin, and the like. In an example, a facial landmark image 304 may be a visual representation that indicates the one or more facial landmark features of the patient in the image 202A of the patient. Further, the facial landmark image 304 may be a two-dimensional (2D) image.

At 306, an inclination data determination operation is performed. In the inclination data determination operation, the processor 104 is configured to determine inclination data associated with the face. The inclination data may be determined based on the image data 202 and the one or more facial landmark features. The inclination data may refer to measurements and observations related to an orientation and a position of the facial structures in relation to the cranial base or other reference points in the face of the patient. The inclination data may be associated with the angles and positions of different parts of the face, particularly how the teeth and jaw are aligned. The inclination data may be utilized to increase the accuracy of identification of the facial landmark features, such as eyes, nose, and mouth. The computer system 102 may utilize the inclination data using facial landmarks to estimate a better initial position of the jaws.

At 308, an oral cavity region identification operation is performed. In the oral cavity region identification operation, the processor 104 is configured to identify an oral cavity region 310 associated with the oral cavity of the patient. The oral cavity region 310 may be identified based on the image data 202, the one or more facial landmark features and the inclination data. In an example, precise oral cavity region 310 is identified based on the one or more facial landmarks associated with the oral cavity. Further, the oral cavity region 310 may be expanded to include pixels associated with, for example, moustache, chin, etc. based on the inclination data. In an example, the oral cavity region 310 may be an image that indicates the oral cavity region and surrounding region in the face of the patient. The oral cavity region 310 may be provided to a first ML Model 312.

The first ML Model 312 may be a computational framework that may be trained to identify patterns in data. The first ML Model 312 uses algorithms to analyse input data and make predictions or decisions based on that analysis. Further, the first ML Model 312 may operate in the identified oral cavity region 310. In an embodiment, the first ML model 312 may utilize the image segmentation algorithm. The image segmentation algorithm may be a computational technique used to partition an image into distinct regions or segments, each of which corresponds to specific objects, features, or areas of interest within the image. In an example, the image segmentation algorithm is utilized to identify and isolate specific features or structures within the image, allowing for further analysis. The first ML model 312 may be utilized to identify different regions associated with the oral cavity such as different dental objects associated with the dental structure.

At 314, a dental structure segmentation operation is performed. In the dental structure segmentation operation, the processor 104 is configured to segment, using the first ML model 312, a dental structure of the oral cavity based on the identified oral cavity region 310. The first ML model 312 may also be configured to segment a gingiva structure of the oral cavity of the patient. The first ML model 312 may apply the image segmentation algorithm on the oral cavity region 310 to segment the dental structure 316 of the oral cavity and the gingiva structure of the oral cavity. The segmentation algorithm works by processing the identified oral cavity region 310 to isolate the dental structure 316, including teeth and their arrangement, as well as the gingiva structure. By effectively segmenting the dental structure 316 and the gingiva structure, the first ML model provides a detailed representation of the oral anatomy of the patient with respect to the image 202A. In an example, a 2D segmented teeth or the dental structure 316 may be a visual representation that indicates teeth component in the oral cavity region 310 of the patient.

At 318, an oral cavity data generation operation is performed. In the oral cavity data generation operation, the processor 104 is configured to generate oral cavity data associated with the oral cavity of the patient. The oral cavity data may correspond to a cropped image of the image 202A that may include the oral cavity region 310. Further, within the cropped image, the dental structure 316 may be segment and highlighted, such as based on different unique colors to generate the oral cavity data. The oral cavity data may indicate locations and values of pixels in the cropped image relating to the dental structure 316 as well as for gingiva structure.

FIG. 4A, FIG. 4B and FIG. 4C illustrate a block diagram of exemplary operations for determining one or more alignment parameters for an upper jaw, in accordance with an example embodiment.

Referring to FIG. 4A, a block diagram 400A of exemplary operations for calculating a first score is illustrated, in accordance with an exemplary embodiment of the present disclosure. FIG. 4A is explained in conjunction with elements of FIG. 1, FIG. 2A, FIG. 2B, and FIG. 3. The first score may be calculated to asses at what stage the alignment of the 3D jaw to the 2D image data is acceptable. The exemplary operations illustrated in the block diagram 400A start at 402 and are performed by any computing system, apparatus, or device, such as by the computer system 102 of FIG. 1. Although illustrated with discrete blocks, the exemplary operations associated with one or more blocks of the block diagram 400A can be divided into additional blocks, combined into fewer blocks, or eliminated, depending on the implementation.

At 402, a pair of consecutive teeth identification operation is performed. In the pair of consecutive teeth identification operation, the processor 104 may be configured to identify a pair of consecutive teeth in each of an image 408 of the oral cavity region 310 and the 3D dental model 106A. The image 408 may correspond to a cropped image of the oral cavity region 310. For example, the cropped image, i.e., the image 408 of the oral cavity region 310 may be generated based on the image 202A and the image data 202 of the face of the patient. Further, the pair of consecutive teeth in the image 408 and the 3D dental model 106A are identified based on identifying consecutive teeth of a predefined type. In an example, the predefined type may be associated with incisors. The incisors may refer to the front teeth in the first portion of the dental structure 316 or the upper jaw in the image 408 and the 3D dental model 106A. The incisors are characterized by their sharp, thin edges, which are designed for cutting and slicing food.

In an embodiment, the processor 104 may identify the pair of consecutive teeth within the image 408 using the oral cavity data 110A. Similarly, the processor 104 may identify the pair of consecutive teeth within the 3D dental model 106A using the set of dental model pixels.

The image 408 may be a virtual representation that represents the dental structure 316. Further, the virtual representation may depict the identified pair of consecutive teeth associated with the dental structure 316. The image 408 may include an incisor "A" and an incisor "B" of the dental structure 316. Similarly, the 3D dental model 408B may be a virtual representation that represents the upper jaw of the patient. The 3D dental model 106A may include an incisor "C" and an incisor "D" of the upper jaw.

In an alternate embodiment, if the incisor of the patient is missing in the image 408, consecutive teeth adjacent to the incisor may be selected. In an example, if the incisor "A" within the image 408 is missing, the computer system 102 may select a tooth adjacent to the incisor "B" placed right to "A".

At 404, an edge data determination operation is performed. In the edge data determination operation, the processor 104 is configured to determine edge data associated with the identified pair of consecutive teeth in the image 408 and the 3D dental model 106A.

At 406, an overlay generation operation is performed. In the overlay generation operation, the processor 104 is configured to generate a first overlay 414 of the 3D dental model 106A on the image 408. Further, the first overlay 414 may be generated based on the edge data.

In an example, the image 408 may be overlaid on the 3D dental model 106A, or vice versa. In the first overlay 414, an edge of the incisor "A" in the image 408 may be aligned with or overlaid on an edge of the incisor "C" in the 3D dental model 106A. The first overlay 414 may be performed such that the incisor "C" may be as close as possible to incisor "A". Similarly, the incisor "D" may be as close as possible to incisor "B".

In an example, the processor 104 may utilize an ML model to perform the transformations needed to be applied for the first overlay 414. The transformations may include translation in X axis, Y axis, and Z axis, and rotation in X axis, Y axis, and Z axis. Further, the ML model may identify the coordinates associated with the consecutive pair of teeth and coordinates of edges of the consecutive pair of teeth to overlay them.

At 410, a transformation function application operation is performed. In the transformation function application operation, the processor 104 is configured to apply a first transformation function to the first overlay 414. The first transformation function is applied to maximize an overlapping surface between the dental structure in the image 408 and the 3D dental model 106A. The first transformation function may maximize the overlapping surface between the dental structure in the image 408 and the 3D dental model 106A.

In an example, the processor 104 may apply the first transformations. Further, the amoeba optimization technique may be utilized to maximize the overlapping surface between the dental structure in the image 408 and the 3D dental model 106A. The amoeba optimization technique, also known as the Nelder-Mead method, is a derivative-free optimization technique that is particularly effective for multidimensional problems where the objective function is not necessarily smooth or differentiable. The amoeba optimization technique iteratively modifies the simplex by reflecting, expanding, contracting, or shrinking it based on the function values at the vertices, allowing it to converge towards an optimal solution. The amoeba optimization technique is especially useful in scenarios where gradient information is unavailable or expensive to compute, making it a valuable tool in various fields, including engineering, economics, and machine learning. By effectively navigating complex landscapes, amoeba optimization aids in finding optimal solutions that meet specific criteria while minimizing computational effort. Further, the operations associated with the amoeba utilization technique are omitted for the sake of brevity.

The amoeba optimization technique may be utilized to maximize the overlapping surface by rotating the digital representation of the upper jaw in the image 408 in X axis, Y axis, or Z axis, or translating the digital representation of the upper jaw in X axis, Y axis, or Z axis. Further, the ML model may identify borders 418A associated with the teeth or the dental structure 316 in the 3D dental model 106A and identify borders 418B of the dental structure in the image 408 using the oral cavity data 110A. The processor 104 may utilize the identified borders 418A and 418B to maximize the overlapping between the dental structure in the image 408 and the upper jaw in the 3D dental model 106A. Further, the maximized overlapping surface may be represented as aligned upper jaw 418.

The aligned upper jaw 418 may be a virtual representation that indicates the maximized overlapping surface between the dental structure in the image 408 and the upper jaw in the 3D dental model 106A. Further, the aligned upper jaw 418, may indicate the identified borders 418A and 418B. Further, the teeth associated with the 3D dental model 106A may be depicted by colored teeth.

At 412, a one or more alignment parameters determination operation is performed. In the one or more alignment parameters determination operation, the processor 104 may be configured to determine the one or more alignment parameters for the 3D dental model 106A based on the application of the first transformation function to the first overlay 414, i.e., the aligned upper jaw 418. The one or more alignment parameters are associated with at least the rotation of the upper jaw of the 3D dental model 106A in at least X axis, Y axis, or Z axis, or a translation of the upper jaw in at least X axis, Y axis, or Z axis.

At 416, a first score calculation operation is performed. In some embodiments, in the first score calculation operation, the processor 104 is configured to calculate a first ratio associated with a maximized overlapping surface or the overlapping surface in the aligned upper jaw 418 between the dental structure in the image 408 and the upper jaw in the 3D dental model 106A. The first ratio may indicate a degree of overlap of the upper jaw on the first dental structure in the image 408 based on the created overlapping between them. In an example, the processor 104 may calculate the first ratio corresponding to "0.75". The first ratio "0.75" indicates that the degree of overlap between the dental structure in the image 408 and the upper jaw in the 3D dental model 106A is approximately "75%".

Referring to FIG. 4B, a block diagram 400B of exemplary operations for calculating a second Score is illustrated, in accordance with an exemplary embodiment of the present disclosure. FIG. 4B is explained in conjunction with elements of FIG. 1, FIG. 2A, FIG. 2B, FIG. 3 and FIG. 4A. The exemplary operations illustrated in the block diagram 400B start at 422 and are performed by any computing system, apparatus, or device, such as by the computer system 102 of FIG. 1. Although illustrated with discrete blocks, the exemplary operations associated with one or more blocks of the block diagram 400B can be divided into additional blocks, combined into fewer blocks, or eliminated, depending on the implementation.

At 422, a centre data determination operation is performed. In the centre data determination operation, the processor 104 is configured to determine centre data associated with each tooth in an image 422A and the 3D dental model 424.

In an embodiment, the processor 104 may receive the image 422A associated with the dental structure of the patient. For example, the image 422A may correspond to the cropped image of the oral cavity region 310 of the patient. In other words, the image 422A may correspond to the oral cavity data 110A of the patient. Further, the 3D dental model 106A may indicate upper jaw of the patient based on a scan of the oral cavity of the patient.

Further, the centre data associated with each tooth of the second upper jaw may be determined using the oral cavity data 110A. In an example, the centre data associated with each tooth of the second upper jaw of the 3D dental model 424 may be identified using a set of dental model pixels.

At 424, a 3D dental model and dental structure overlay generation operation is performed. In the 3D dental model and dental structure overlay generation operation, the processor 104 is configured to generate a second overlay 424A of the dental structure identified from the image 422A and the 3D dental model 106A. The second overlay is generated based on the centre data. In an example, the edges or boundary of the dental structure identified from the image 422A is aligned with the 3D dental model 106A. The alignment is performed by identifying a centre of each tooth in the 3D dental model 106A and the image 422A. For example, for a tooth, a centre of the tooth in the 3D dental model 106A is aligned with the corresponding centre of the tooth in the image 422A.

The second overlay 424A may be a virtual representation that indicates an overlay of the edge of the dental structure, i.e., teeth or tooth, on the 3D dental model 106A. Further, the centre of each tooth in the dental structure of the image 422A is identified using the oral cavity data 110A. Similarly, the centre of each tooth in the 3D dental model 106A is identified using the set of dental model pixels. At the end, the centre of each tooth in the dental structure and the centre of each tooth in the upper jaw of the 3D dental model 106A are utilized to generate the second overlay 424A.

At 426, a transformation function application operation is performed. In the transformation function application operation, the processor 104 is configured to apply the first transformation function, or a second transformation function to the second overlay 424A. The second transformation function may be applied to minimize a distance between corresponding centres for each tooth in the dental structure and the 3D dental model 106A.

In an embodiment, the processor 104 may utilize an amoeba optimization technique for the second transformation. Further, the second transformation may be associated with the translation of the upper jaw in the 3D dental model 106A in X, Y, and Z axis, or the rotation of the second upper jaw in X, Y, and Z axis. Using the amoeba optimization technique the distance between the corresponding centres for each tooth in the dental structure and the upper jaw may be minimized.

Further, the processor 104 apply the first transformation function based on the application of the second transformation function. The first transformation function is applied for maximizing an overlapping surface between the dental structure in the image 422A and the upper jaw in the 3D dental model 106A. The operations associated with application of the first transformation function is like the operations described, for example, at 410 in FIG. 4A.

Based on the application of the first transformation function and/or the second transformation function to the second overlay 424A, an aligned upper jaw 426A may be generated. The aligned upper jaw 426A may be a visual representation indicating the aligned upper jaw of the 3D dental model 106A with the dental structure in the cropped image 422A indicating the oral cavity region 310.

At 428, a one or more alignment parameters determination operation is performed. In the one or more alignment parameters determination operation, the processor 104 may be configured to determine the one or more alignment parameters for alignment of the 3D dental model 106A with the dental structure of the patient in the image 422A based on the application of the first transformation function and/or the second transformation function to the second overlay 424A. In an example, the one or more alignment parameters are associated with at least a rotation of the upper jaw in the 3D dental model 106A in at least X axis, Y axis, or Z axis, or the translation of the upper jaw in at least X axis, Y axis, or Z axis.

At 430, a second score calculation operation is performed. In the second score calculation operation, the processor 104 may be configured to determine a second ratio associated with the overlapping surface in the aligned upper jaw 426A. The second ratio may indicate a degree of overlap of the upper jaw on the dental structure. In an example, the processor 104 may calculate the second ratio corresponding to the "0.81". The second ratio "0.81" indicates that the degree of overlap of the upper jaw of the 3D dental model 106A and the upper jaw of the patient in the dental structure is approximately "81%".

FIG. 4C illustrates a block diagram 400C for identifying the one or more alignment parameters, in accordance with an exemplary embodiment of the present disclosure. FIG. 4C is explained in conjunction with elements of FIG. 4A and FIG. 4B. The exemplary operations illustrated in the block diagram 400C start at 432 and are performed by any computing system, apparatus, or device, such as by the computer system 102 of FIG. 1.

At 422, the first score and the second score are received. The first score is associated with the first overlay 414 or the aligned upper jaw 418. Similarly, the second score is associated with the second overlay 424A or the aligned upper jaw 426A. The processor 104 may receive the first score and the second score from the memory of the computer system 102. In an example, the processor 104 may receive the first score as a ratio defined as "0.75" and the second score as a second ratio as "0.81".

At 444, the first ratio and the second ratio are compared. The processor 104 may compare the first ratio and the second ratio to identify a bigger value amongst the first ratio and the second ratio. In an example, the processor 104 may compare the first ratio of "0.75" and the second ratio of "0.81". Based on the comparison the processor 104 may identify an optimum ratio corresponding to correspond to the second ratio, i.e., "0.81". The optimum ratio of "0.81" may be associated with maximized overlapping surface between the upper jaw in the dental structure in the image 408 or 422A and the upper jaw in the 3D dental model 106A.

At 446, the one or more alignment parameters are identified. The processor 104 may identify the one or more alignment parameters that is associated with the optimum ratio. In an example, the processor 104 may identify the one or more alignment parameters associated with the second ratio of "0.81". The one or more alignment parameters is associated with at least the rotation of the upper jaw in at least X axis, Y axis, or Z axis, or a translation of the upper jaw in at least X axis, Y axis, or Z axis. In an embodiment, the one or more alignment parameters may be associated with the values that represents the coordinates of the aligned upper jaw.

FIG. 5A illustrate a block diagram 500A of exemplary operations for determining the one or more alignment parameters based on a bite type, in accordance with an example embodiment. With reference to FIG. 5A there is shown a first dental image 502, a second dental image 504, a third dental image 506, an aligned open bite image 512, and an aligned close bite image 514. FIG. 5A is explained in conjunction with elements of FIG. 1, FIG. 2A, FIG. 2B, FIG. 3, FIG. 4A, FIG. 4B, and FIG. 4C.

In an embodiment, the processor 104 may be configured to receive one of the first dental image 502, the second dental image 504, or the third dental image 506. Each of the first dental image 502, the second dental image 504, and the third dental image 506 may correspond to the cropped image of the oral cavity region 310. The cropped image may indicate the oral cavity data 110A. For example, the first dental image 502, the second dental image 504, and the third dental image 506 are example embodiments of the image 408 or the image 422A.

The first dental image 502 may be a virtual representation that indicates the oral cavity region 310. Further, the first dental image 502 may be associated with an open bite type. The open bite type may be indicative of a gap between an upper jaw and a lower jaw in a dental structure of a patient. Further, the second dental image 504 may be a virtual representation of the oral cavity region 310 that is associated with a closed bite type. The closed bite type may indicate that there is no gap between the upper jaw and the lower jaw in a dental structure of a patient. Further, the third dental image 506 may be a virtual representation of the oral cavity region 310 that is associated with a no lower bite type. The no lower bite type may indicate that a lower jaw of the patient is not visible in a dental structure of a patient.

At 508, a bite type determination operation is performed. In the bite type determination operation, the processor 104 is configured to determine a bite type associated with the one of the first dental image 502, the second dental image 504, or the third dental image 506. In an embodiment, the processor 104 may receive the oral cavity data 110A associated with the dental structure of the patient. Further, the processor 104 may determine the bite type based on the oral cavity data 110A. In an example, the processor 104 may determine the bite type corresponding to the open bite type, closed bite, or no lower bite type.

At 510, a one or more alignment parameters determination operation is performed. In the one or more alignment parameters determination operation, the processor 104 may determine the one or more alignment parameters associated with an alignment of the 3D dental model 106A with the dental structure of the patient based on the bite type.

In an example, a manner in which the one or more alignment parameters of the 3D dental model 106A are determined is dependent on a bite type. In an example, the one or more alignment parameters may be determined based on the application of the first transformation function or the second transformation function. It may be noted that operations for determining the one or more alignment parameters for aligning the upper jaw of the 3D dental model 106A with upper jaw in the dental structure of the patient in the image of the oral cavity region 310 are described in conjunction with FIG. 4A, FIG. 4B, and FIG. 4C.

Further, the processor 104 may determine one or more alignment parameters for aligning a lower jaw of the 3D dental model 106A with lower jaw in the dental structure of the patient in the image of the oral cavity region 310 based on the determined bite type. Details associated with the operations for determining the one or more alignment parameters for different bite types of the lower jaw are described in conjunction with, for example, FIG. 5B and FIG. 5C.

In particular, operations associated with determining the one or more alignment parameters for a patient with the open bite type are explained in conjunction with FIG. 5B. Further, operations associated with determining the one or more alignment parameters for a patient with the closed bite type are explained in conjunction with FIG. 5C. Further, in an embodiment, when a patient has a no lower bite type, then the one or more alignment parameters are associated with only the upper jaw. Determining the alignment parameters for the upper jaw has been explained in conjunction with FIG. 4A, FIG. 4B and FIG. 4C.

In an embodiment, the processor 104 may align the upper jaw and the lower jaw of the 3D dental model 106A with upper jaw and the lower jaw on the dental structure of the oral cavity region 310 separately based on the determined one or more alignment parameters when the bite type of the patient is open bite type. Such an alignment for the open bite type can be visualized using the aligned open bite 512. The aligned open bite 512 may be a virtual representation that indicates the aligned upper jaw and the lower jaw of the 3D dental model 106A with the dental structure.

In an alternate example, the processor 104 may align the upper jaw and the lower jaw of the 3D dental model with the upper jaw and the lower jaw in the dental structure of the patient together or as a whole when the bite type of the patient is close bite type. The alignment can be visualized using the aligned close bite 514. The aligned close bite 514 may be a virtual representation that indicates the aligned upper jaw and the lower jaw of the 3D dental model 106A with the dental structure.

FIG. 5B illustrates a block diagram 500B of exemplary operations for determining the one or more alignment parameters associated with an open bite type, in accordance with an example embodiment. With reference to FIG. 5B there is shown a first image 516, the one or more alignment parameters 520A, and an aligned lower jaw 526. FIG. 5B is explained in conjunction with elements of FIG. 1, FIG. 2A, FIG. 2B, FIG. 3, FIG. 4A, FIG. 4B, FIG. 4C, and FIG. 5A. Further, the one or more alignment parameters 520A may include upper jaw alignment parameters 522 and lower jaw alignment parameters 524.

In an embodiment, the processor 104 may be configured to receive the first image 516 may be an exemplary implementation of the image 408 or the image 422A. The first image 516 may indicate the oral cavity region 310 of the patient. The oral cavity region would include the dental structure of the patient and the gingiva structure of the patient. Further, the processor 104 may receive the first image 516 from the memory of the computer system 102.

At 518, a bite type determination operation may be performed. In the bite type determination operation, the processor 104 may determine the bite type associate with the first image 516. In an example, the processor 104 may determine the bite type of the first image 516 corresponding to the "open bite type". The processor 104 may determine the bite type based on the oral cavity data 110A associated with the first image 516and information associated with predefined bite types.

At 520, a one or more alignment parameters determination operation is performed. In an example, the processor 104 may determine the one or more alignment parameters 520A associated with the open bite type. The one or more alignment parameters 520A may be associated with at least the rotation of a portion of the 3D dental model 106A in the at least X axis, Y axis, or Z axis, or the translation of the portion of the 3D dental model 106A in the at least X axis, Y axis, or Z axis. The alignment parameters may also comprise a determination of the field of view of the virtual camera. The portion may correspond to the upper jaw in one case and to lower jaw in another case. To this end, the processor 104 may determine the upper jaw alignment parameters 522 and the lower jaw alignment parameters 524 as part of the one or more alignment parameters 520A. For example, the upper jaw alignment parameters 522 are determined first for the upper jaw, for example, based on the operations described in conjunction with FIG. 4A, FIG. 4B and FIG. 4C. Thereafter, the same operations are applied for determining the lower jaw alignment parameters 524 associated with the lower jaw of the 3D dental model 106A. The processor 104 may utilize the first transformation function and/or the second transformation function on the lower jaw to determine the set of lower jaw alignment parameters 524.

In an embodiment, initially, the processor 104 may determine centre data associated with each tooth in the lower jaw of the dental structure and the lower jaw in the 3D dental model 106A based on the oral cavity data 110A and the set of dental model pixels. Further, the processor 104 may generate an overlay of each tooth of the lower jaw of the dental structure over each tooth of the lower jaw in the 3D dental model 106A. Further, the processor 104 may apply the second transformation function on the lower jaw of the identified open bite type to minimize the distance in the overlay. Further, the processor 104 may determine the lower jaw alignment parameters 524 based on the transformation. The set of lower jaw alignment parameters 522 may be associated with the rotation of the lower jaw in at least X axis or the translation of the lower jaw in at least X axis and Y axis. The rotation in Y axis and Z axis, and translation in Z axis is not considered due to the movements of the lower jaw being physically conditioned by the upper jaw. In an example, the lower jaw only translates in X axis and Y axis.

Further, the processor 104 may apply the first transformation function based on the second transformation function. The first transformation function may be applied to maximize an overlapping area. Further, the processor 104 may determine the lower jaw alignment parameters 524 associated with the application of the first transformation function. The operations associated with the first transformation function and the second transformation function are explained in conjunction with FIG. 4A, FIG. 4B, and FIG. 4C.

At the end, the processor 104 may determine the set of lower jaw alignment parameters 524 associated with both the first transformation function and the second transformation function. Further, the processor 104 may align the lower jaw of the 3D dental model 106A with the lower jaw of the dental structure based on the lower jaw alignment parameters 524. Further, the alignment may be visualized using the aligned lower jaw 526.

The aligned lower jaw 526 may be a virtual representation that indicates the aligned lower jaw based on the application of the first transformation and/or the second transformation. Further, the aligned lower jaw 526 may indicate an overlap of each tooth of the lower jaw in the 3D dental model 106A on each tooth of lower jaw in the dental structure of the patient.

FIG. 5C illustrates a block diagram 500C of exemplary operations for determining the one or more alignment parameters associated with a close bite type, in accordance with an example embodiment. With reference to FIG. 5C there is shown a second image 528, and an aligned jaw 534. FIG. 5C is explained in conjunction with elements of FIG. 1, FIG. 2A, FIG. 2B, FIG. 3, FIG. 4A, FIG. 4B, FIG. 4C, FIG. 5A and FIG. 5B.

In an embodiment, the processor 104 may be configured to receive the second image 528. The second image 528 may be a virtual representation that indicates the oral cavity region 310. Further, the processor 104 may receive the second image 528 from the memory of the computer system 102. In an example, the second image 528 may correspond to the "closed bite type".

At 530, a bite type determination operation may be performed. In the bite type determination operation, the processor 104 may determine the bite type associate with the second image 528. In an example, the processor 104 may determine the bite type of the second image 528 corresponding to the "closed bite type". In an embodiment, the processor 104 may execute the bite type determination operation similar to the bite type determination operation 518.

At 520, a one or more alignment parameters determination operation is performed. In an example, the processor 104 may determine the one or more alignment parameters 532A associated with the closed bite type. In an embodiment, the processor 104 may consider the upper jaw and the lower jaw as a unified entity for determining the one or more alignment parameters 532A for the closed bite type. Further, the operations performed for determining the one or more alignment parameters 532A may correspond to the operations described in the FIG. 4A, FIG. 4B and FIG. 4C for determining alignment parameters for upper jaw.

Further, the processor 104 may align the upper jaw and the lower jaw of the 3D dental model 106A with the upper jaw and the lower jaw of dental structure based on the one or more alignment parameters 532A associated with the unified entity. Further, the alignment may be visualized using the aligned jaw 534. The aligned jaw 534 may be a virtual representation indicating the aligned upper and lower jaw on the dental structure associated with the closed bite type. The aligned jaw 534 may indicate an overlap of each tooth of the upper jaw and the lower jaw in the 3D dental model 106A on each tooth of the dental structure.

FIG. 6A and FIG. 6B collectively illustrate a block diagram for generating a background image of a patient, in accordance with an example embodiment. Referring to FIG. 6A, a block diagram 600A for generating a part of the visualization model 110D is illustrated, in accordance with an exemplary embodiment of the present disclosure. With reference to FIG. 6A, there is shown a 2D segmented teeth 602, the oral cavity region 310, and a segmented image 606. FIG. 6A is explained in conjunction with elements of FIG. 1, FIG. 2A, FIG. 2B, FIG. 3, FIG. 4A, FIG. 4B, FIG. 4C, FIG. 5A, FIG. 5B, and FIG. 5C. Further, the segmented image 606 may include a first set of pixels 606A. Further, the 2D segmented teeth 602 may be an example embodiment of the 2D segmented teeth 316.

In an embodiment, the processor 104 may receive the 2D segmented teeth 602 and the image data 202. Further, the alignment may be visualized using the aligned lower jaw 534. The aligned lower jaw 534 may be a virtual representation indicating the aligned lower jaw on the dental structure associated with the closed bite type. The aligned lower jaw 534 may indicate an overlap of each tooth of the lower jaw on each tooth of the dental structure. The processor 104 may receive the 2D segmented teeth 602 and the image data 202 from the memory of the computer system 102.

At 604, a first segmented image generation operation is performed. In the first segmented image generation operation, the processor 104 may be configured to generate a first segmented image associated with the oral cavity. The first segmented image may be generated based on the 2D segmented teeth 602 and the image data 202. The first segmented image may include the first set of pixels 606A associated with the dental structure and a second set of pixels associated with at least the gingiva structure. The second set of pixels may also be associated with other oral structures which are not the teeth, such as the tongue, inner cheek, etc. The first set of pixels 606A may be identified based on the 2D segmented teeth 602. Further, the second set of pixels may be identified based on the 2D segmented teeth 602 and the image data 202. Specifically, the processor 104 may apply image processing techniques to identify the second set of pixels. The image processing techniques refer to a set of methods and algorithms used to manipulate and analyse images. The image processing techniques may be applied to digital images to extract useful information or prepare them for further analysis or interpretation.

Further, a value of each pixel of the first set of pixels 606A is set to a predefined value in the first segmented image. In an example, the predefined value may correspond to "black". The processor 104 may identify the first set of pixels 606A of the oral cavity region 310 based on the 2D segmented teeth 602. The processor 104 may set each pixel of the first set of pixels 606A of the oral cavity region 310 to the predefined value "black". Further, the first segmented image may be visualized using the first segmented image 606. The segmented image 606 may be a visual representation of the first segmented image. The segmented image 606 may indicate the first set of pixels 606A corresponding to "black".

At 606, a first pixel identification operation is performed. In the first pixel identification operation, the processor 104 may be configured to identify a first pixel from the first set of pixels 606A. The first pixel is having at least one first adjacent pixel. In some embodiments, the adjacent pixels may be considered to be pixels in a geometric shape centered around the pixel under consideration. For example, the adjacent pixels may be defined as the pixels in a square of a certain size centered around the pixel under consideration. The size of number of pixels considered to be adjacent, may be only the closes pixels directly touching the pixel under consideration, or may be defined as all pixels within a certain distance from the pixel under consideration. The at least one first adjacent pixel is associated with the second set of pixels. In an embodiment, the first pixel may be randomly selected from the first set of pixels 606A.

At 610, a first color average determination operation is performed. In first color average determination operation, the processor 104 may be configured to determine a first color average associated with the at least one first adjacent pixel based on a first pixel value associated with each of the at least one first adjacent pixel. In an example, the processor 104 may identify each of a first adjacent pixel, a second adjacent pixel, a third adjacent pixel and, a fourth adjacent pixel of the at least one first adjacent pixel. Further, the processor 104 may identify the pixel value associated with each of the first adjacent pixel, the second adjacent pixel, the third adjacent pixel, and the fourth adjacent pixel. Furthermore, the processor 104 may determine an average of the pixel value associated with each of the first adjacent pixel, the second adjacent pixel, the third adjacent pixel, and the fourth adjacent pixel. The average value may correspond to the first color average associated with the first pixel from the first set of pixels 606A. Similarly, the processor 104 may identify the first color average associated with each pixel from the first set of pixels 606A that have at least one adjacent pixel in the second set. This may be done for example by selecting a pixel from the first set of pixels, finding the average color, applying noise to the selected pixel and moving the pixel from the first set of pixels to the second set of pixels.

At 612, a first normally distributed noise addition operation is performed. In the first normally distributed noise addition operation, the processor 104 may be configured to add a first normally distributed noise to the first color average. The first normally distributed noise distribution, or gaussian noise distribution may be a statistically distributed noise characterized by a bell-shaped probability density function (PDF). The PDF may be a statistical function that describes the likelihood of a continuous random variable taking on a particular value.

In an embodiment, the processor 104 may generate the first distribution of the first color average. In an example, the first distribution may be a normal distribution. The normal distribution, also known as gaussian distribution, may be a continuous probability distribution that may be symmetric around its mean, forming a bell-shaped curve, where most values are close to the mean, and probabilities decrease as values move further away. Further, the processor 104 may add the first normally distributed noise to decrease the intensity of the first color average of the first distribution.

At 614, a first corrected pixel value generation operation is performed. In the oral cavity data generation operation, the processor 104 may be configured to generate a first corrected pixel value of the set of pixel values 110C based on the addition. In an embodiment, the processor 104 may generate a corrected pixel value of the set of pixel values 110C corresponding to the decreased intensity of the first color average.

At 616, a first pixel adjustment operation is performed. In the first pixel adjustment operation, the processor 104 may be configured to adjust the first pixel based on the first corrected pixel value. In an embodiment, the processor 104 may set the first pixel of the first set of pixels 606A to the corrected pixel value of the set of pixel values 110C to adjust the first pixel. Similarly, the processor may adjust each pixel of the first set of pixels 606A, if, once the pixel is adjusted, it is added to the second set of pixels.

At 616, a part of visualization model generation operation is performed. In the part of visualization model generation operation, the processor 104 may be configured to generate at least a part of the visualization model based on the adjusted first pixel. The processor 104 may generate a first part of the visualization model corresponding to the at least the part of the visualization model. The first part of visualization model may be associated with the dental structure. The first part of visualization model may correspond to the adjusted first set of pixels 606A.

FIG. 6B illustrates a block diagram 600B of exemplary operations for generating the background image, in accordance with an exemplary embodiment of the present disclosure. With reference to FIG. 6B there is shown a first segmented image 606, and the oral cavity region 310. FIG. 6B is explained in conjunction with elements of FIG. 6A.

In an embodiment, the processor 104 may receive the first segmented image and the image data 202. The image data may be associated with the image of the patient. The processor 104 may receive the first segmented image and the image data 202 from the memory of the computer system 102.

At 620, a second segmented image generation operation is performed. In the second segmented image generation operation, the processor 104 may be configured to generate a second segmented image 622 associated with the oral cavity. The second segmented image may be generated based on the first segmented image 606 and the image data 202. The second segmented image 622 may include the first set of pixels 606A associated with the dental structure and a second set of pixels 622A associated with the gingiva structure. The second set of pixels may be identified based on the first segmented image 606 and oral cavity data 110A associated with the oral cavity. A value of each pixel of the second set of pixels 622A is set to the predefined value, such as a value corresponding to black color in the second segmented image 622. In an example, the processor 104 may set each pixel of the second set of pixels 622A of the oral cavity to the predefined value "black".

At 624, a second pixel identification operation is performed. In the second pixel identification operation, the processor 104 may be configured to identify a second pixel from the second set of pixels 622A. The second pixel is having at least one second adjacent pixel. The at least one second adjacent pixel is associated with the first set of pixels 606A. In an embodiment, the second pixel may be randomly selected from the second set of pixels 622A.

At 626, a second color average determination operation is performed. In second color average determination operation, the processor 104 may be configured to determine a second color average associated with the at least one second adjacent pixel. The second color average may be determined based on a second pixel value associated with each of the at least one second adjacent pixel. Further, the operation associated with the second color average determination may be similar to the first color average determination operation 610.

At 628, a second normally distributed noise addition operation is performed. In the second normally distributed noise addition operation, the processor 104 may be configured to add a second normally distributed noise to a second distribution of the second color average. Further, the operation associated with the second normally distributed noise addition may be similar to the first normally distributed noise addition operation 610.

At 630, a second corrected pixel value generation operation is performed. In the oral cavity data generation operation, the processor 104 may be configured to generate a second corrected pixel value of the set of pixel values 110C based on the addition. Further, the operation associated with the second corrected pixel value generation may be similar to the first corrected pixel value generation operation 610.

At 632, a second pixel adjustment operation is performed. In the second pixel adjustment operation, the processor 104 may be configured to adjust the second pixel based on the second corrected pixel value. In an embodiment, the processor 104 may set the second pixel of the second set of pixels to the second corrected pixel value of the set of pixel values 110C to adjust the second pixel. Similarly, the processor 104 may adjust each pixel of the second set of pixels. Further, the operation associated with the first pixel adjustment may be similar to the first pixel adjustment operation 610.

At 634, a part of visualization model generation operation is performed. In the part of visualization model generation operation, the processor 104 may be configured to generate at least a part of the visualization model associated with the gingiva structure. The part of the visualization model may be generated based on the adjusted second pixel. The part of visualization model may be associated with the adjusted second set of pixels 622B. The first part of visualization model may be associated with the gingival structure.

At 636, a background image generation operation is performed. In the background image generation operation, the processor 104 may be configured to generate the background image associated with the oral cavity. The processor 104 may utilize the second part of the visualization model and the first part of visualization model to generate the background image. In an example, the background image is a combination of the second part of the visualization model and the first part of visualization model. In an example, the background image is visualized in a portion of background image 904.

Alternatively, or in addition to the workflow described above, the method may comprise:
- Selecting a pixel from the first (black) set that has at least one adjacent pixel in the second set. The selecting may be random.
- Calculating the average color of these adjacent pixels.
- Adding normally distributed noise to the selected pixel.
- Removing the selected pixel from the first set and adding it to the second set.
- Repeating all steps above until the first set is empty, i.e. when all black pixels have been given a color value.

FIG. 7A and FIG. 7B illustrate a block diagram of exemplary operations for updating a 3D template gingiva model, in accordance with an example embodiment. Referring to FIG. 7A, a block diagram 700A for updating colors of the 3D template gingiva model 106B is illustrated, in accordance with an exemplary embodiment of the present disclosure. With reference to FIG. 7A there is shown 3D template gingiva model 106B, a patient image 712, color map data 716, and a current gingiva image 720. Further, the patient image 712 may include a first portion 712A. FIG. 7A is explained in conjunction with elements of FIG. 1, FIG. 2A, FIG. 2B, FIG. 3, FIG 4A, FIG 4B, FIG 4C, FIG 5A, FIG 5B, FIG. 5C, FIG. 6A and FIG. 6B.

In an embodiment processor 104 may receive the image data 202 associated with the patient. The image data 202 may be received from the memory of the computer system 102. Further, the oral cavity data 110A may be received associated with the image data 202. The oral cavity data 110A may include the information derived from the oral cavity, such as a comprehensive collection of information related to the anatomical, physiological, and pathological aspects of the oral cavity, which may include structures such as the lips, cheeks, gums, teeth, tongue, and palates. In an example, the oral cavity data 110A may include information associated with the set of gingiva pixels associated with the gingiva structure.

At 702, a set of gingiva pixels identification operation is performed. In the set of gingiva pixels identification operation, the processor 104 may be configured to identify a set of gingiva pixels associated with the gingiva structure 704. The processor 104 may identify the set of gingiva pixels based on the oral cavity data 110A and the image data 202 associated with the gingiva structure 704. In an example, the processor 104 may identify a pixel "68, 14, 11" of the set of gingiva pixels. The pixel "68, 14, 11" may be associated with a dark shade of red. The set of identified gingiva pixels may be visualized using the gingiva pixels. The gingiva pixels may be a virtual representation indicating the set of gingiva pixels of the patient.

At 706, a 3D template gingiva model retrieval operation is performed. In the 3D template gingiva model retrieval operation, the processor 104 may be configured to retrieve the 3D template gingiva model 106B. The 3D template gingiva model 106B may be retrieved from the database 106. The set of template gingival pixels of the 3D template gingiva model 106B is distributed across a coloring coordinate. The coloring coordinate may be associated with each pixel of the set of gingiva pixels. Further, each gingiva pixels of the set of gingiva pixels are associated with unique colors. In an example, the template gingiva pixel corresponding to "209,0,132" of the set of template gingival pixels may correspond to "68, 14, 11". Further "209,0,132" may represent a unique color corresponding to "a shade of purple".

Further, the unique colors associated with the set of template gingiva pixels are distributed across each of a horizontal plane and a vertical plane of the 3D template gingiva model 106B. In an example, shades of red and green color are distributed across the vertical plane. Further, different blue shades are spread across the horizontal plane of the 3D template gingiva model 106B. The unique colors associated with the set of template gingiva pixels may be visualized using the template gingiva model 106B. The template gingiva model 106B may be a virtual representation indicating the set of template gingiva pixels of the patient. Further, the template gingiva model 106B may indicate the unique colors associated with the set of template gingiva pixels.

In an embodiment, each pixel of the set of template gingiva pixels which may correspond to a pixel of the set of gingiva pixels is assigned a unique color. Based on this, the 3D template gingiva model 106B is updated. The remaining portions associated with the 3D template gingiva model 106B are identified and removed. Further, the updated 3D template gingiva model 106B may be visualized using the template gingiva model image 708.

At 710, a patient image generation operation is performed. In the patient image generation operation, the processor 104 may be configured to generate the patient image 712. The patent image may be generated based on masking the set of gingiva pixels with the set of template gingival pixels. The masking is done by replacing the set of gingiva pixels from the image with the set of template gingiva pixels. In an embodiment, the processor 104 may align the identified 3D template gingiva model 106B with the gingiva structure 704 using the set of gingiva pixels and the set of template gingival pixels. The alignment may mask the set of gingiva pixels from the image with the updated set of template gingiva pixels. In an example, the processor 104 may replace the first gingiva pixel corresponding to "68, 14, 12" with the first template gingiva pixel "209,0.132".

Further, the patient image may be visualized using the patient image 712. The patient image 712 may be a virtual representation that indicates the masking of the set of gingiva pixels with the set of template gingival pixels. The first portion 712A of the patient image 712 may be indicative of the unique colors.

At 714, a color map data generation operation is performed. In the color map data generation operation, the processor 104 may be configured to generate color map data for each pixel of the set of template gingival pixels based on a value of a corresponding pixel of the set of gingival pixels in the gingiva structure 704. The color map data may include mapping of each template gingiva pixel of the set of template gingiva pixels with the value associated with the corresponding set of gingiva pixels. The value may indicate color associated with a pixel of the set of gingival pixels. In an example, the value may correspond to "68, 14, 11" which may indicate the dark shade of red. Further, the processor 104 may store the color map data in the memory of the computer system 102.

The color map data may be visualized using a color map table 716. The color map table 716 may be a tabular representation that indicates the mapping associated with the color of the set of template gingiva pixels with the set of gingiva pixels. In an example, the first template gingiva pixel "209,0.132" is mapped with the value "68, 14, 11".

Further, the computer system 102 may utilize the mapping to identify the one or more intensity parameters 110B. The one or more intensity parameters 110B may be associated with the value of the set of gingival pixels. In an example, an intensity parameter of the one or more intensity parameters 110B may correspond to the value. Further, the computer system 102 may identify the one or more intensity parameters 110B based on the occurrence of a template gingiva pixel in the mapping. If no template gingiva pixel may be found, this may indicate that there may be no value corresponding to it may be present in the mapping. Further, the processor 104 may identify the one or more adjacent pixels of the selected template gingiva pixel and take the average of their values.

At 718, a 3D template gingiva model update operation is performed. In the 3D template gingiva model update operation, the processor 104 may be configured to update colors of the 3D template gingiva model 106B based on the one or more intensity parameters 110B. The processor 104 may identify the value associated with each pixel of the set of gingiva pixels using each template gingiva pixel of the set of template gingiva pixels from the color map data. The processor 104 may update the color of each template gingiva pixel of the set of template gingiva pixels based on the value to generate the current gingiva model. Further, the current gingiva model may be visualized using the current gingiva image 720. The current gingiva image 720 may be a virtual representation that indicates the current gingiva model.

Referring to FIG. 7B, there is shown a block diagram 700B for updating the colors of the 3D template gingiva model based on the one or more visualization attributes, in accordance with an exemplary embodiment of the present disclosure. With reference to FIG. 7B there is shown a visualized gingiva image 728. FIG. 7B is explained in conjunction with elements of FIG. 1, FIG. 2A, FIG. 2B, FIG. 3, FIG 4A, FIG 4B, FIG 4C, FIG 5A, FIG 5B, FIG. 5C, FIG. 6A, FIG. 6B and FIG. 7A.

In an embodiment, the processor 104 may receive the color map data 722 associated with the current gingiva model. The color map data 722 may include mapping of each template gingiva pixel of the set of template gingiva pixels with a value of color or pixel associated with the corresponding pixel from the set of gingiva pixels. The processor 104 may receive the color map data 722 from the memory of the computer system 102.

Further, the processor 104 may receive one or more visualization attributes associated with the operative process. The operative process may be associated with the modification of the oral cavity. The operative process may include teeth whiting, veneers, gingival depigmentation, orthodontic applications, retainers, prophylaxis, dental sealants, and the like. In an example, the patient may want to perform the gingival depigmentation. The gingival depigmentation may be performed to lighten dark gums, often caused by excess melanin. The user may perform the gingival depigmentation operation using the patient image to lighten patient gingiva. The patient gingiva is an exemplary embodiment of the gingiva structure 704. The user may utilise different dental operations to perform the gingival depigmentation operation to update colors of the patient gingiva. The updated colors of the patient gingiva may correspond to the one or more visualization attributes associated with the gingival depigmentation operation.

At 724, a visualization attributes application operation is performed. In the visualization attributes application operation, the processor 104 may be configured to apply the one or more visualization attributes on the color map data associated with the gingiva structure 704. In an example, the processor 104 may update the mapping associated with the color map data based on the one or more visualization attributes. Subsequently, updated color map data is generated. The processor 104 may map the updated colors of the patient gingiva with the set of template gingiva pixel. Further based on the mapping the one or more intensity parameters 110B may be determined.

At 726, a 3D template gingiva model update operation is performed. In the 3D template gingiva model update operation, the processor 104 may be configured to update colors of the 3D template gingiva model 106B based on updated color map data. The processor 104 may update the color of each template gingiva pixel of the set of template gingiva pixel based on the updated color map data to generate the visualized gingiva model. 728.

FIG. 8 illustrates a block diagram 800 of exemplary operations for generating visualized dental structure of the patient, in accordance with an exemplary embodiment of the present disclosure. With reference to FIG. 8 there is shown a brightness curve 810, an editing slider 816, and a visualized dental model image 824. Further, the visualized dental model image 824 may include a portion of visualized dental model 824. FIG. 8 is explained in conjunction with elements of FIG. 1.

In an embodiment, the processor 104 may receive the image data 202 associated with the patient. The image data 202 may be received from the memory of the computer system 102. Further, the oral cavity data 110A may be received associated with the image data 202. The oral cavity data 110A may include the information associated with the dental structure of the patient. In an example, the oral cavity data 110A may include information associated with the set of dental pixels associated with the dental structure.

At 802, a set of dental pixels identification operation is performed. In the set of dental pixels identification operation, the processor 104 may be configured to identify the set of dental pixels associated with a dental structure 804. The processor 104 may identify the set of dental pixels based on the oral cavity data 110A associated with the dental structure 804. In an example, the processor 104 may identify pixel "150, 75, 0" of the set of dental pixels. The pixel "150, 75, 0" may correspond to a shade of brown. Further, the set of dental pixels may be visualized using the dental structure 804. The dental structure 804 may be a virtual representation that indicates the set of dental pixels. The dental structure 804 may a model, such as a 3D model of the teeth associated with the dental structure 804 containing only the set of dental pixels.

At 806, an intensity data determination operation is performed. In the intensity data determination operation, the processor 104 may be configured to determine intensity data associated with the set of dental pixels. The intensity data is associated with a brightness level of each column of pixels of the set of dental pixels. In an embodiment, the processor 104 may identify an average brightness of colors associated with each column of pixels of the set of dental pixels. The average brightness may correspond to the brightness level. In an example, the width of the column may correspond to "1 pixel". Further, the height of the column may correspond to "n pixels" where n may be greater than or equal to "1". In an example, the height of the column may be associated with the height of the teeth in the dental pixel image 804. Alternatively, the intensity data, instead of being associated with the colors associated with each column of pixels as described above, the intensity data may be associated with an average brightness level of a set of pixels representing at least a part of a tooth.

At 808, a brightness level normalization operation is performed. In the brightness level normalization operation, the processor 104 may be configured to normalise the brightness level of each column of pixels. The brightness level is normalized based on a predefined range. In an example, the predefined range may correspond to "0 to 1". The processor 104 may normalize the brightness value between "0 to 1".

At 812, an intensity curve generation operation is performed. In the intensity curve generation operation, the processor 104 may be configured to generate an intensity curve. The intensity curve is generated based on the normalization. The intensity curve may indicate the brightness value associated with each column of pixels of the set of dental pixels.

In an example, the processor 104 may arrange each column of pixels of the set of dental pixels on the X axis of the brightness curve and the corresponding brightness value on the Y-axis of the brightness curve. In an example, the processor 104 may arrange each column of pixels of the set of dental pixels from the right tooth in the dental pixel image 804 to the left tooth in the dental pixel image 804 on the X axis. Further, the intensity curve may be visualized using the brightness curve 810. The brightness curve 810 may be a virtual representation that indicates the intensity curve over the dental pixel image 804. The brightness curve 810 may indicate the brightness level of each column associated with the dental pixel image 804.

Further, the processor 104 may remove an effect of shadow associated with each tooth of the dental structure 804. The effect of shadow may be associated with the level of brightness of the lightning source present during the image generation process. In an embodiment, the processor 104 may apply the moving average technique on the intensity curve to remove the effect of shadows. In the moving average technique, the processor 104 may determine a smoothing window associated with the brightness values of the intensity curve. In an example, the processor may determine the window of "4 alternate brightness value". For the moving average calculation, the processor 104 may calculate the sum of the brightness levels of the smoothing window. Further, the processor 104 may divide the sum by the length of the smoothing window.

Further, the processor 104 may approximate the brightness curve. The processor 104 may approximate the brightness curve as a set of non-overlapping line segments. The set of line segments is the distance between each pair of stop values. The stop values may correspond to a column of pixels of the set of dental pixels. Initially, the processor 104 may determine the stop values which are at equal distances from each other in the x-axis of the brightness curve. Further, the processor 104 may apply a linear least square approximation on each pair of stop values associated with each of the line segments to generate the best line segment which may fit the corresponding brightness level of the intensity curve. The linear least square approximation may be a statistical method used to find the best-fitting linear function corresponding to a line segment that approximates a set of data points. The data points may correspond to the brightness level of each column of pixels of the set of dental pixels between a pair of stop values. Details associated with the linear least square approximation are omitted for the sake of brevity.

Further, the processor 104 may minimize the approximation. The processor 104 may utilize simulated annealing to minimize the approximation. The simulated annealing may be a stochastic optimization technique that approximates the global minimum of a cost function .. The simulated annealing may be utilized to minimize the sum of squared residual (R^2). The residual may be associated with the column of pixels of the set of dental pixels in the brightness curve. The residual may be a distance between a line segment that corresponds to a particular column of pixels and the actual brightness value associated with that column. The simulated annealing may be an iterative approach, where in each iteration, a new state is found by randomly moving the stop values of each line segment to the left or right. The new state may be accepted with a probability that depends on the R^2 measure of the new state, the R^2 measure of the previous state, and the number of iterations that have passed. Further, the processor 104 may identify the stop points associated with the minimized approximation. Details associated with the stimulated annealing are omitted for the sake of brevity. In other embodiments, instead of an average brightness of colors associated with each column of pixels of the set of dental pixels, the average brightness level of colors may be associated with an average brightness level of a set of pixels representing at least a part of a tooth, or a whole tooth. The rest of the method would in this case proceed as described above.

At 814, a set of points identification operation is performed. In the set of points identification operation, the processor 104 may be configured to identify a set of points extending along a predefined direction across the dental structure 804. Further, the set of points may be identified based on stochastic optimization. The set of points may correspond to the stop values identified after the minimized approximation. In an example, the predefined direction may correspond to "left to right" direction on the x-axis or "right to left" direction on the x-axis. Further, the processor 104 may store the identified set of points in the memory of the computer system 102.

Further, the set of points may be visualized using the editing slider 816. The editing slider 816 may be a virtual representation that indicates the intensity curve over the editing slider 816. The editing slider 816 may indicate a first points 816A-816F. In an example, the first points 816A-816F may correspond to the set of points.

At 818, a current dental model generation operation is performed. In the current dental model generation operation, the processor 104 may be configured to generate the current dental model based on the set of identified points. The processor 104 may utilize a gradient shader to generate the current dental model generation. The gradient shader may be a type of shading technique used in computer graphics to create smooth transitions between colors or shades across a surface. The gradient shader is commonly employed in rendering techniques to enhance the visual appearance of objects by simulating the effects of light and color blending. In an embodiment, the processor 104 may feed the set of points to the gradient shader. The gradient shader may apply the corresponding brightness level associated with the set of points on the 3D dental model 106A to generate the current dental structure. In an example, the gradient shader may take the set of points along the predefined direction of "left to right" with their corresponding brightness value. Further, the gradient shader may apply a linearly increasing or decreasing darkening between the set of points on the 3D dental model 106A to generate the current dental model.

At 820, a one or more visualization attributes application operation is performed. In the one or more visualization attributes application operation, the processor 104 may be configured to apply the one or more visualization attributes to one or more pixels of the set of dental pixels lying between each consecutive point of the set of points. The one or more visualization attributes are applied based on the brightness level associated with the one or more pixels indicated by the intensity curve.

In an embodiment, the processor 104 may receive one or more visualization attributes associated with the operative process. In an example, the operative process may correspond to teeth whitening. The teeth whiting may be performed to lighten the teeth of the dental structure of the patient. The user may perform the teeth whitening operation using the patient image to brighten the teeth of the patient. The user may utilise different dental operations on the image of the patient to perform the teeth whitening operation to generate the modified colors associated with the dental structure of the patient in the image. The modified colors associated with the dental structure may correspond to the one or more visualization attributes associated with the teeth whitening operation.

At 822, visualized dental model generation operation is performed. In the post-dental model generation operation, the processor 104 may be configured to generate the visualized dental model based on the one or more visualization attributes and the set of points.

In an example, the processor 104 may apply the one or more visualization attributes associated with the teeth whitening on the set of points to update the brightness level associated with the set of points. Further, the processor 104 may utilize the gradient shader to generate the visualized dental model. The visualized dental model may be visualized using visualized dental model image 824. The visualized dental model image 824 may be a virtual representation that indicates the visualized dental model. The portion of visualized dental model 824A may correspond to the visualized dental model.

In an example, the visualized dental model is a 3D dental model.

FIG. 9A illustrates a block diagram 900A of exemplary operations for generating a current image of the patient, in accordance with an exemplary embodiment of the present disclosure. With reference to FIG. 9A there is shown a portion of background image 904, and a current image 912. FIG. 9A is explained in conjunction with elements of FIG. 1, FIG. 2A, FIG. 2B, FIG. 3, FIG. 4A, FIG. 4B, FIG. 4C, FIG. 5A, FIG. 5B, FIG.5C, FIG.6A, FIG.6B, FIG.7A, FIG.7B, and FIG.8.

At 902, a background image retrieval operation is performed. In the background image retrieval operation, the processor 104 may be configured to generate the background image associated with the face of the patient. The background image is generated based on the image data 202. The background image comprises one or more facial characteristics of the face. The one or more facial characteristics are exclusive of the oral cavity. Further, the operations associated with the generation of the background image are described in conjunction with FIG. 6A, and FIG. 6B.

The oral cavity associated with the background image may be visualized using the portion of background image 904. The portion of background image 904 may be a virtual representation that indicates the first part of visualization model and the second part of visualization model.

At 906, a current visualization model generation operation is performed. In current visualization model generation operation, the processor 104 may be configured to generate a current visualization model of the oral cavity based on the 3D dental model 106A, the 3D template gingiva model 106B, the one or more alignment parameters 524, the one or more intensity parameters 110B. The processor 104 may generate the current dental model based on the 3D dental model 106A. Similarly, the processor 104 may generate the current gingiva model based on the 3D template gingiva model 106B. The current dental model and the current gingiva model may be the part of the current visualization model. Further, the processor 104 may store the current visualization model in the memory of the computer system 102. Further, the operations associated with the current visualization model generation are further described in conjunction with FIG. 1, FIG. 2A, FIG. 2B, FIG. 3, FIG. 4A, FIG. 4B, FIG. 4C, FIG. 5A, FIG. 5B, FIG.5C, FIG.6A, FIG.6B, FIG.7A, FIG.7B, and FIG.8.

At 908, a current visualization model overlay is performed. In the current visualization model overlay operation, the processor 104 may be configured to overlay the current visualization model on the background image. In an embodiment, the processor 104 may overlay the current visualization model on the background image based on the one or more alignment parameters 524.

In an alternate embodiment, the processor 104 may overlay the current visualization model on the background image based on the first set of pixels associated with the dental structure of the background image, the second set of pixels associated with the gingiva structure 704 of the background image, the set of dental model pixels, and the set of template gingiva pixels. Further, the first set of pixels and the second set of pixels may be associated with coordinates of the background image. Similarly, the set of dental model pixels and the set of template gingiva pixels may be associated with coordinates of the current visualization model.

At 910, a current image generation operation is performed. In the current image generation operation, the processor 104 may be configured to generate a current image 912 based on the overlay. The current image 912 indicates the patient of their actual status of their oral cavity before the operative process. The current image 912 may inform the patient about the actual health of their oral cavity. The current image 912 may be a virtual representation that represents the oral cavity of the current image.

FIG. 9B illustrates a block diagram 900B of exemplary operations for generating an update image of the patient, in accordance with an exemplary embodiment of the present disclosure. With reference to FIG. 9B there is shown an updated image 924. FIG. 9B is explained in conjunction with elements of FIG. 9A.

In an embodiment processor 104 may receive the current visualization model. Further, the processor 104 may receive the color map data and the set of points associated with current dental model. The current visualization model, the color map data, and the set of points may be received or retrieved from the memory of the computer system 102.

At 914, a dental structure and gingiva structure segmentation operation is performed. In the dental structure and gingiva structure segmentation operation, the processor 104 may be configured to segment each of the dental structure of the oral cavity and the gingiva structure of the oral cavity based on the overlay. The processor 104 may generate segmented data associated with the dental structure and gingiva structure based on the segmentation. In an example, the segmented data may include information associated with the set of dental model pixels and the set of template gingiva pixels.

At 916, a one or more visualization attributes application operation is performed. In the one or more visualization attributes application operation, the processor 104 may be configured to apply one or more visualization attributes on the current visualization model based on the segmented data. The processor 104 may apply the one or more visualization attributes associated with the operative process to update the set of dental model pixels and the set of template gingiva pixels. The processor 104 may apply the one or more visualization attributes associated with the operative process to update the set of dental model pixels and the set of template gingiva pixels.

At 918, a visualization model generation operation is performed. In the visualization model generation operation, the processor 104 may be configured to generate the visualization model of the oral cavity based on the segmentation and the set of pixel values 110C associated with the 3D dental model 106A. Further, the operations associated with the visualization model generation are further described in conjunction with FIG. 1, FIG. 2A, FIG. 2B, FIG. 3, FIG. 4A, FIG. 4B, FIG. 4C, FIG. 5A, FIG. 5B, FIG.5C, FIG.6A, FIG.6B, FIG.7A, FIG.7B, and FIG.8.

At 920, a visualization model overlay operation is performed. In the visualization model overlay operation, the processor 104 may be configured to overlay the visualization model on the background image. Further, the operation associated with visualization model overlay may be similar to the current visualization model overlay 908.

At 922, an updated image generation operation is performed. In the updated image generation operation, the processor 104 may be configured to generate the updated image 924 of the face of the patient based on the overlaying and the image data 202. The updated image 924 may represent modification to the oral cavity of the patient.

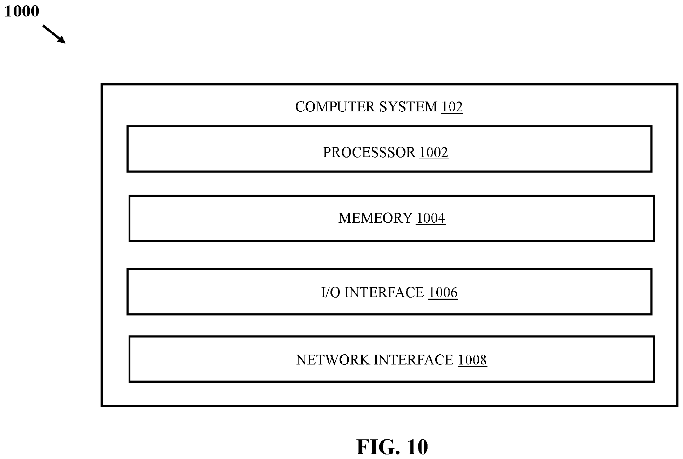

FIG. 10 illustrates a block diagram 1000 of the computer system 102 for generating the visualization model 110D of the oral cavity data of the patient, in accordance with an example embodiment. FIG. 10 is explained in conjunction with elements of FIG. 1- FIG. 9B. The computer system 102 may include one or more processors (hereinafter, also referred to as a processor 1002), a memory unit 1004, an input/output (I/O) unit 1006, and a network interface 1008.

The processor 1002 may be embodied in a number of different ways. For example, the processor 1002 may be embodied as one or more of various hardware processing means such as a coprocessor, a microprocessor, a controller, a digital signal processor (DSP), a processing element with or without an accompanying DSP, or various other processing circuitry including integrated circuits such as, for example, an ASIC (application specific integrated circuit), an FPGA (field programmable gate array), a microcontroller unit (MCU), a hardware accelerator, a special-purpose computer chip, or the like. In an embodiment, the processor 1002 may be embodied as a high-performance microprocessor having series of System on Chip (SOCs) which may include relative powerful and power-efficient Graphics Processing Units (GPUs) and Central Processing Units (CPUs) and a small form factor. As such, in some embodiments, the processor 1002 may include one or more processing cores configured to perform independently. A multi-core processor may enable multiprocessing within a single physical package. Additionally, or alternatively, the processor 1002 may include one or more processors configured in tandem via the bus to enable independent execution of instructions, pipelining and/or multithreading.

In some embodiments, the processor 1002 may be configured to detect NIR and visible light, using the I/O interface 1006 during the scanning session of teeth of a subject, such as a patient requiring a dental treatment. The detected NIR and visible light may be used to generate images of the patient, such as the image 202A. The image 202A may include images of dental structure or teeth of the patient from a viewing point. Further, the processor 1002 is configured to generate the visualization model 110D of the oral cavity of the patient. In an example, based on the visualization model, the processor 1002 is further configured to generate updated image of the patient. For example, the updated image may indicate a modification or transformation of a feature of the oral cavity of the patient post an operative process. Such operative process may include, but is not limited to, teeth whitening, teeth contouring, and so forth.

In an example embodiment, the processor 1002 may be in communication with the memory unit 1004 via a bus for passing information.

The memory unit 1004 may be non-transitory and may include, for example, one or more volatile and/or non-volatile memories. In other words, for example, the memory unit 1004 may be an electronic storage device (for example, a computer readable storage medium) comprising gates configured to store data (for example, bits) that may be retrievable by a machine (for example, a computing device like the processor 1002). The memory unit 1004 may be configured to store information, data, content, applications, instructions, or the like, for enabling the apparatus to carry out various functions in accordance with an example embodiment of the present disclosure. For example, the memory unit 1004 may be configured to store the image 202A. As exemplarily illustrated in FIG. 10, the memory unit 1004 may be configured to store instructions for execution by the processor 1002. As such, whether configured by hardware or software methods, or by a combination thereof, processor 1002 may represent an entity (for example, physically embodied in circuitry) capable of performing operations according to an embodiment of the present disclosure while configured accordingly. Thus, for example, when the processor 1002 is embodied as the microprocessor, the processor 1002 may be specifically configured hardware for conducting the operations described herein. Alternatively, as another example, when the processor 1002 is embodied as an executor of software instructions, the instructions may specifically configure the processor 1002 to perform the algorithms and/or operations described herein when the instructions are executed. The processor 1002 may include, among other things, a clock, an arithmetic logic unit (ALU) and logic gates configured to support operation of the processor 1002.

The I/O interface 1006 may include circuitry and/or software that may be configured to provide output to the user of the computer system 102 and receive, measure or sense input information. The I/O interface 1006 may include a speaker, a vibrator, a projector unit, and one or more sensors.

The network interface 1008 may comprise input interface and output interface for supporting communications to and from the computer system 102. The network interface 1008 may be a device or circuitry embodied in either hardware or a combination of hardware and software that is configured to receive and/or transmit data to/from the computer system 102. In this regard, the network interface 1008 may include, for example, an antenna (or multiple antennae) and supporting hardware and/or software for enabling communications with a wireless communication channel. Additionally, or alternatively, the network interface 1008 may include the circuitry for interacting with the antenna(s) to cause transmission of signals via the antenna(s) or to handle receipt of signals received via the antenna(s). In some environments, the network interface 1008 may alternatively or additionally support wired communication. As such, for example, the network interface 1008 may include a communication modem and/or other hardware and/or software for supporting communication via cable, digital subscriber line (DSL), universal serial bus (USB) or other mechanisms.

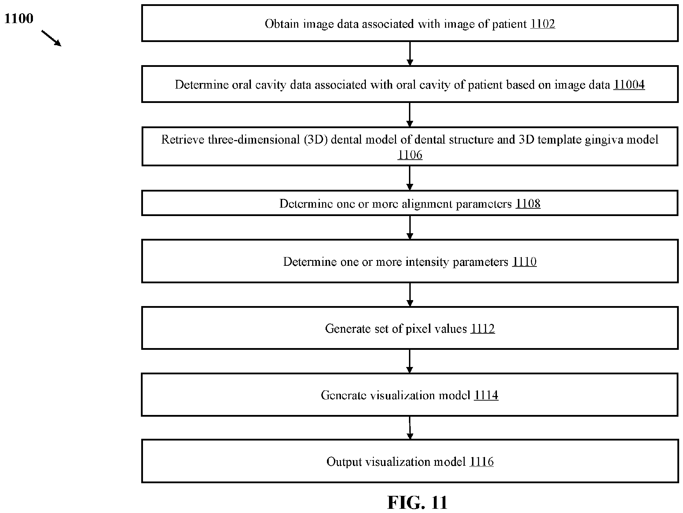

FIG. 11 illustrates a flowchart 1100 of a method for generating the visualization model 110D of the oral cavity of the patient, in accordance with an example embodiment. FIG. 11 is explained in conjunction with elements of FIG. 1, FIG. 2A, FIG. 2B, FIG. 3, FIG. 4A, FIG. 4B, FIG. 4C, FIG. 5A, FIG. 5B, FIG.5C, FIG.6A, FIG.6B, FIG.7A, FIG.7B, FIG.8, FIG.9A, FIG.9B, FIG. 10, and FIG. 11. The operations of the exemplary method may be executed by any computing system, for example, by the processors 104 of the computer system 102 of FIG. 1. The operations of the flowchart 1100 may start at 1102.

At 1102, the image data 202 associated with the image of the patient may be obtained. In an embodiment, the computer system 102 is configured to obtain the image data 202 associated with the image of the patient. Further, the image data 202 is indicative of the face of the patient. Details associated with the obtaining of the image data 202 are provided, for example, in FIG. 1, FIG. 2, FIG. 3, FIG. 4, FIG. 5, FIG. 6A, FIG. 6B, FIG. 7A, and FIG. 7B.

At 1104, the oral cavity data 110A may be determined. In an embodiment, the computer system 102 is configured to determine oral cavity data 110A associated with an oral cavity of the patient based on the image data. The oral cavity data is associated with the dental structure of the oral cavity and the gingiva structure of the oral cavity. Details associated with the determination of oral cavity data 110A are provided, for example, in FIG. 1, FIG. 2, FIG. 3, FIG. 4, FIG. 5, FIG. 6A, FIG. 6B, FIG. 7A, and FIG. 7B.

At 1106, the 3D dental model 106A of the dental structure and the 3D template gingiva model 106B may be retrieved. In an embodiment, the computer system 102 is configured to retrieve the 3D dental model 106A of the dental structure and the 3D template gingiva model 106B. Details associated with the retrieval of the 3D dental model 106A and 3D template gingiva model 106B are provided, for example, in FIG. 1, FIG. 2, FIG. 3, FIG. 4, FIG. 5, FIG. 6A, FIG. 6B, FIG. 7A, and FIG. 7B.

At 1108, the one or more alignment parameters 524 may be determined. In an embodiment, the computer system 102 is configured to determine the one or more alignment parameters 524 for aligning at least one of the first portion of the 3D dental model 106A, or the second portion of the 3D dental model 106A with the dental structure of the oral cavity based on the oral cavity data 110A. Details associated with the determination of the one or more alignment parameters 524 are provided, for example, in FIG. 1, FIG. 2, FIG. 3, FIG. 4, FIG. 5, FIG. 6A, FIG. 6B, FIG. 7A, and FIG. 7B.

At 1110, the one or more intensity parameters 110B may be determined. In an embodiment, the computer system 102 is configured to determine one or more intensity parameters 110B for matching the first set of colors of the 3D template gingiva model 106B with the gingiva structure, based on the oral cavity data. Details associated with the determination of the one or more intensity parameters 110B are provided, for example, in FIG. 1, FIG. 2, FIG. 3, FIG. 4, FIG. 5, FIG. 6A, FIG. 6B, FIG. 7A, and FIG. 7B.

At 1112, the set of pixel values 110C may be generated. In an embodiment, the computer system 102 is configured to generate the set of pixel values 110C for correcting the second set of colors of the 3D dental model 106A based on the oral cavity data and one or more visualization attributes. The one or more visualization attributes are associated with the operative process for the modification of the oral cavity. Details associated with the generation of the set of pixel values 110C are provided, for example, in FIG. 1, FIG. 2, FIG. 3, FIG. 4, FIG. 5, FIG. 6A, FIG. 6B, FIG. 7A, and FIG. 7B.

At 1114, the visualization model 110D may be generated. In an embodiment, the computer system 102 is configured to generate the visualization model 110D for the oral cavity of the patient based on the 3D dental model 106A, the 3D template gingiva model 106B, the one or more alignment parameters 524, the one or more intensity parameters 110B, and the set of pixel values 110C. Details associated with the generation of the visualization model are provided, for example, in FIG. 1, FIG. 2, FIG. 3, FIG. 4, FIG. 5, FIG. 6A, FIG. 6B, FIG. 7A, and FIG. 7B.

At 1116, the visualization model 110D for oral cavity may be outputted. In an embodiment, the computer system 102 is configured to output the visualization model 110D for the oral cavity. Details associated with the output of the visualization model 110D are provided, for example, in FIG. 1, FIG. 2, FIG. 3, FIG. 4, FIG. 5, FIG. 6A, FIG. 6B, FIG. 7A, and FIG. 7B.

Accordingly, blocks of the flowchart 1100 support combinations of means for performing the specified functions and combinations of operations for performing the specified functions. It will also be understood that one or more blocks of the flowchart 1100 can be implemented by special purpose hardware-based computer systems which perform the specified functions, or combinations of special purpose hardware and computer instructions.

Alternatively, the scanner device 104 may include means for performing each of the operations described above. In this regard, according to an example embodiment, examples of means for performing operations may include, for example, a processor and/or a device or circuit for executing instructions, such as the operations or instructions for controlling heat transfer in the scanner device 104.

Many modifications and other embodiments of the disclosure set forth herein will come to mind of one skilled in the art to which these disclosures pertain having the benefit of the teachings presented in the foregoing descriptions and the associated drawings. Therefore, it is to be understood that the disclosures are not to be limited to the specific embodiments disclosed and that modifications and other embodiments are intended to be included within the scope of the appended claims. Moreover, although the foregoing descriptions and the associated drawings describe example embodiments in the context of certain example combinations of elements and/or functions, it should be appreciated that different combinations of elements and/or functions may be provided by alternative embodiments without departing from the scope of the appended claims. In this regard, for example, different combinations of elements and/or functions than those explicitly described above are also contemplated as may be set forth in some of the appended claims. Although specific terms are employed herein, they are used in a generic and descriptive sense only and not for purposes of limitation.

## Claims

1. A computer implemented method (1100) for generating a visualization model (110D) of an oral cavity, the method comprising:
obtaining (1102) image data (202) associated with an image (202A) of a patient, wherein the image data (202) is indicative of a face of the patient;
determining (1104) oral cavity data (110A) associated with an oral cavity of the patient based on the image data, wherein the oral cavity data is associated with a dental structure (804) of the oral cavity and a gingiva structure (704) of the oral cavity;
retrieving (1106) a three-dimensional (3D) dental model (106A) of the dental structure and a 3D template gingiva model (106B);
determining (1108) one or more alignment parameters (520A, 532A) for aligning at least one of: a first portion of the 3D dental model, or a second portion of the 3D dental model (106A) with the dental structure of the oral cavity based on the oral cavity data;
determining (1110) one or more intensity parameters (110B) for matching a first set of colors of the 3D template gingiva model with the gingiva structure based on the oral cavity data;
generating (1112) a set of pixel values (110C) for a second set of colors of the 3D dental model based on the oral cavity data and one or more visualization attributes, wherein the one or more visualization attributes are associated with a manipulation of the oral cavity in the image of the patient;
generating (1114) a visualization model (110D) for the oral cavity of the patient based on the 3D dental model (106A), the 3D template gingiva model (106B), the one or more alignment parameters (524), the one or more intensity parameters (110A), and the set of pixel values (110C); and
outputting (1116) the visualization model (110D) for the oral cavity.

2. The method according to claim 1, further comprising:
identifying (302) one or more facial landmark features associated with the face of the patient based on the image data (202);
determining (306) inclination data associated with the face based on the image data and the one or more facial landmark features;
identifying (308) an oral cavity region (310) associated with the oral cavity of the patient based on the image data, the one or more facial landmark features and the inclination data;
segmenting, using a first machine learning (ML) model (312), the dental structure (804) of the oral cavity and the gingiva structure (704) of the oral cavity based on the identified oral cavity region (310); and
generating (318) the oral cavity data (110A) based on the segmentation.

3. The method according to any one of the claims 1 or 2, further comprising:
obtaining scan data associated with an intraoral cavity of the patient;
generating (222), using a second ML model (220), the 3D dental model (106A) of the dental structure (804) of the patient; and
storing (324) the 3D dental model of the dental structure.

4. The method according to any one of the preceding claims, further comprising:
generating (636) a background image associated with the face of the patient based on the image data (202), wherein the background image comprises one or more facial characteristics of the face, and wherein the one or more facial characteristics are exclusive of the oral cavity;
generating (906) a current visualization model of the oral cavity based on the 3D dental model (106A), the 3D template gingiva model (106B), the one or more alignment parameters (520A, 532A), and the one or more intensity parameters (110B);
overlaying (908) the current visualization model on the background image;
segmenting (914) each of the dental structure (804) of the oral cavity and the gingiva structure (704) of the oral cavity based on the overlay;
generating (918) the visualization model (110D) of the oral cavity based on the segmentation and the set of pixel values (110C) associated with the 3D dental model (106A);
overlaying (920) the visualization model on the background image; and
generating (922) an updated image (924) of the face of the patient based on the overlaying and the image data (202).

5. The method according to claim 4, further comprising:
generating (604) a first segmented image (606) associated with the oral cavity, the first segmented image comprising a first set of pixels (606A) associated with the dental structure (804) and a second set of pixels associated with the gingiva structure (704), and wherein a value of each pixel of the first set of pixels is set to a predefined value in the first segmented image;
identifying (610) a first pixel from the first set of pixels, the first pixel having at least one first adjacent pixel, wherein the at least one first adjacent pixel is associated with the second set of pixels;
determining (608) a first color average associated with the at least one first adjacent pixel based on a first pixel value associated with each of the at least one first adjacent pixel;
adding (612) a first normally distributed noise to a first distribution of the first color average;
generating (614) a first corrected pixel value of the set of pixel values (110C) based on the addition;
adjusting (616) the first pixel based on the first corrected pixel value; and
generating (618) at least a part of the visualization model based on the adjusted first pixel.

6. The method according to any one of claims 4 or 5, further comprising:
generating (620) a second segmented image (622) associated with the oral cavity, the second segmented image comprising the first set of pixels associated with the dental structure (804) and the second set of pixels associated with the gingiva structure (704), and wherein a value of each pixel of the second set of pixels is set to the predefined value in the second segmented image;
identifying (624) a second pixel from the second set of pixels, the second pixel having at least one second adjacent pixel, wherein the at least one second adjacent pixel is associated with the first set of pixels;
determining (626) a second color average associated with the at least one second adjacent pixel based on a second pixel value associated with each of the at least one second adjacent pixel;
adding (628) a second normally distributed noise to a second distribution of the second color average;
generating (630) a second corrected pixel value of the set of pixel values (110C) based on the addition;
adjusting (632) the second pixel based on the second corrected pixel value; and
generating (634) at least a part of the visualization model based on the adjusted second pixel.

7. The method according to any one of the preceding claims, wherein determining the one or more intensity parameters (110B) further comprises:
identifying (702) a set of gingiva pixels associated with the gingiva structure (704) based on the oral cavity data (110A);
retrieving (706) the 3D template gingiva model (106B), wherein a set of template gingival pixels of the 3D template gingiva model is distributed across a coloring coordinate;
generating (708) a patient image (712) based on masking the set of gingiva pixels with the set of template gingival pixels; and
generating (710) color map data (716) for each pixel of the set of template gingival pixels based on a value of a corresponding pixel of the set of gingival pixels.

8. The method according to any one of the preceding claims, wherein generating the set of pixel values (110C) further comprises:
identifying (802) a set of dental pixels associated with the dental structure (804) based on the oral cavity data;
determining (806) intensity data associated with the set of dental pixels, wherein the intensity data is associated with a brightness level of each column of pixels of the set of dental pixels;
normalizing (808) the brightness level of each column of pixels based on a predefined range;
generating (812) an intensity curve based on the normalization;
identifying (814) a set of points extending along a predefined direction across the dental structure; and
applying (820) the one or more visualization attributes to one or more pixels of the set of dental pixels lying between each consecutive point of the set of points, wherein the one or more visualization attributes are applied based on brightness value associated with the one or more pixels indicated by the intensity curve.

9. The method according to any one of the preceding claims, wherein the first portion of the 3D dental model (106A) is associated with an upper jaw and the second portion of the 3D dental model (106A) is associated with a lower jaw.

10. The method according to claim 9, further comprising
identifying (402) a pair of consecutive teeth in each of the image (408) and the 3D dental model (106A), wherein the pair of consecutive teeth is associated with a predefined type;
determining (404) edge data associated with the pair of consecutive teeth in each of the image and the 3D dental model (106A);
determining centre data associated with each tooth in each of the image and the 3D dental model;
generating a first overlay of the 3D dental model on the dental structure (804) of the image based on the edge data;
generating a second overlay of the 3D dental model on the dental structure of the image based on the centre data; and
applying a first transformation function to the first overlay, wherein the first transformation function is applied for maximizing an overlapping surface between the dental structure and the 3D dental model;
applying at least one of: the first transformation function, or a second transformation function to the second overlay, wherein the second transformation function is applied for minimizing a distance between corresponding centres for each tooth in the dental structure and the 3D dental model; and
determining the one or more alignment parameters (520A, 532A) for the 3D dental model based on the application of first transformation function to the first overlay, or the application of at least one of: the first transformation function, or a second transformation function to the second overlay.

11. The method according to any one of the preceding claims, wherein further comprises:
identifying a bite type associated with the oral cavity based on the image data (202) and a set of predefined bite types; and
determining (510, 520, 532) the one or more alignment parameters (520A, 532A) based on the identified bite type.

12. The method according to any one of the preceding claims, wherein the one or more alignment parameters (520A, 532A) are associated with at least one of: a rotation of a portion of the 3D dental model (106A) in at least one of: X axis, Y axis, or Z axis, or a translation of the portion of the 3D dental model in at least one of: X axis, Y axis, or Z axis, and wherein the portion of the 3D dental model corresponds to one of the first portion or the second portion.

13. A computer system (102) for generating a visualization model (110D) of an oral cavity, the computer system comprising:
a memory (1004) configured to store computer executable instructions; and
one or more processors (1002) configured to execute the computer executable instructions to:
obtain image data (202) associated with an image (202A) of a patient, wherein the image data (202) is indicative of a face of the patient;
determine oral cavity data (110A) associated with an oral cavity of the patient based on the image data, wherein the oral cavity data is associated with a dental structure (804) of the oral cavity and a gingiva structure (704) of the oral cavity;
retrieve a three-dimensional (3D) dental model (106A) of the dental structure and a 3D template gingiva model (106B);
determine one or more alignment parameters (520A, 532A) for aligning at least one of: a first portion of the 3D dental model, or a second portion of the 3D dental model with the dental structure of the oral cavity based on the oral cavity data;
determine one or more intensity parameters (110B) for matching a first set of colors of the 3D template gingiva model with the gingiva structure, based on the oral cavity data;
generate a set of pixel values (110C) for a second set of colors of the 3D dental model, based on the oral cavity data and one or more visualization attributes, the one or more visualization attributes are associated with a manipulation of the oral cavity in the image of the patient;
generate a visualization model (110D) for the oral cavity of the patient based on the 3D dental model, the 3D template gingiva model, the one or more alignment parameters, the one or more intensity parameters, and the set of pixel values; and
output the visualization model for the oral cavity.

14. The computer system (102) according to claim 13, wherein the one or more processors (1002) are further configured to:
identify a set of dental pixels associated with the dental structure (804) based on the oral cavity data (110A);
determine intensity data associated with the set of dental pixels, wherein the intensity data is associated with a brightness level of each column of pixels of the set of dental pixels;
normalize the brightness level of each column of pixels based on a predefined range;
generate an intensity curve based on the normalization;
identify a set of points extending along a predefined direction across the dental structure; and
apply the one or more visualization attributes to one or more pixels of the set of dental pixels lying between each consecutive point of the set of points, wherein the one or more visualization attributes are applied based on brightness value associated with the one or more pixels indicated by the intensity curve.

15. A computer program product comprising instructions which, when executed by a computer, cause the computer to carry out steps of:
obtaining (1102) image data (202) associated with an image (202A) of a patient, wherein the image data is indicative of a face of the patient;
determining (1104) oral cavity data (110A) associated with an oral cavity of the patient based on the image data, wherein the oral cavity data is associated with a dental structure (804) of the oral cavity and a gingiva structure (704) of the oral cavity;
retrieving (1106) a three-dimensional (3D) dental model (106A) of the dental structure and a 3D template gingiva model (106B);
determining (1108) one or more alignment parameters (520A, 532A) for aligning at least one of: a first portion of the 3D dental model, or a second portion of the 3D dental model with the dental structure of the oral cavity based on the oral cavity data;
determining (1110) one or more intensity parameters (110B) for matching a first set of colors of the 3D template gingiva model with the gingiva structure, based on the oral cavity data;
generating (1112) a set of pixel values (110C) for a second set of colors of the 3D dental model based on the oral cavity data and one or more visualization attributes, wherein the one or more visualization attributes are associated with a manipulation of the oral cavity in the image of the patient;
generating (1114) a visualization model (110D) for the oral cavity of the patient based on the 3D dental model, the 3D template gingiva model, the one or more alignment parameters, the one or more intensity parameters, and the set of pixel values; and
outputting (1116) the visualization model for the oral cavity.
